(19) **Europäisches Patentamt European Patent Office Office européen des brevets**

(11) **EP 2 646 776 B1**

(12) **FASCICULE DE BREVET EUROPEEN**

(45) Date de publication et mention de la délivrance du brevet:
**06.09.2017 Bulletin 2017/36**

(21) Numéro de dépôt: **11805080.6**

(22) Date de dépôt: **01.12.2011**

(51) Int Cl.:
*G01C 21/00* (2006.01)  *G01C 21/20* (2006.01)
*G01C 21/16* (2006.01)  *G01C 22/00* (2006.01)
*A61B 5/11* (2006.01)

(86) Numéro de dépôt international:
**PCT/FR2011/052844**

(87) Numéro de publication internationale:
**WO 2012/072961 (07.06.2012 Gazette 2012/23)**

(54) **PROCEDE ET SYSTEME DE DETERMINATION DE VALEURS DE PARAMETRES REPRESENTATIFS D'UN MOUVEMENT D'AU MOINS DEUX MEMBRES D'UNE ENTITE REPRESENTEE SOUS LA FORME D'UNE CHAINE ARTICULEE**

VERFAHREN UND SYSTEM ZUR BESTIMMUNG DER WERTE DER CHARAKTERISTISCHEN PARAMETER EINER BEWEGUNG VON MINDESTENS ZWEI GLIEDMASSEN EINER EINHEIT IN FORM EINER GELENKIGEN LEITUNG

METHOD AND SYSTEM FOR DETERMINING THE VALUES OF PARAMETERS REPRESENTATIVE OF A MOVEMENT OF AT LEAST TWO LIMBS OF AN ENTITY REPRESENTED IN THE FORM OF AN ARTICULATED LINE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.12.2010  FR 1059965**
**01.12.2010  FR 1059967**

(43) Date de publication de la demande:
**09.10.2013  Bulletin 2013/41**

(73) Titulaires:
• **Commissariat à l'Énergie Atomique et aux Énergies Alternatives**
**75015 Paris (FR)**
• **Movea**
**38000 Grenoble (FR)**

(72) Inventeurs:
• **YOUSSEF, Joe**
**F-38000 Grenoble (FR)**
• **GODIN, Christelle**
**F-38190 Brignoud (FR)**
• **LESECQ, Suzanne**
**F-38190 Froges (FR)**

(74) Mandataire: **Bonnet, Michel**
**Cabinet Bonnet**
**93, rue Réaumur - Boîte 10**
**75002 Paris (FR)**

(56) Documents cités:
**EP-A1- 1 970 005        EP-A1- 1 990 138**
**WO-A2-2007/093641**

• **DEJNABADI HOOMAN ET AL: "Estimation and Visualization of Sagital Kinematics of Lower Limbs Orientation Using Body-Fixed Sensors", IEEE TRANSACTIONS ON BIOMEDICAL ENGINEERING, IEEE SERVICE CENTER, PISCATAWAY, NJ, USA, vol. 53, no. 7, 1 juillet 2006 (2006-07-01), pages 1385-1393, XP002475932, ISSN: 0018-9294, DOI: 10.1109/TBME.2006.873678**

**Description**

L'invention porte sur un système de détermination de valeurs de paramètres représentatifs d'un mouvement d'au moins deux membres d'une entité adaptée pour être représentée sous la forme d'une chaîne articulée. Ce mouvement a pour but d'engendrer un déplacement de ladite entité.

Une chaîne articulée est composée de segments rigides sensiblement rectilignes et d'articulations.

Les paramètres représentatifs d'un mouvement d'une telle entité peuvent être des paramètres d'orientation (ou position angulaire) et de position (ou position cartésienne).

Le déplacement de l'entité peut être, par exemple, pour une chaîne articulée représentant des membres inférieurs d'un être vivant ou artificiel (par exemple robotisé), un déplacement par marche, un déplacement par course, un déplacement par saut, ou un déplacement à vélo. Pour une chaîne articulée représentant des membres supérieurs, le déplacement de l'entité peut être, par exemple, un déplacement par actionnement ou simulation d'actionnement de rames, d'aviron ou de pagaies, sur un canoë, un kayak, ou un rameur.

Dans le cas d'un déplacement d'une chaîne articulée, le mouvement de la chaîne articulée est caractérisé par l'orientation et/ou la position d'un point des segments de la chaîne articulée au cours du temps, ou tout au moins à des instants particuliers.

Par exemple, l'estimation de la position de l'ensemble d'une chaîne articulée peut servir à la navigation. Un exemple de dispositif de navigation est le système GPS, pour "Global Positioning System" en langue anglaise, qui est aujourd'hui largement utilisé. Cependant le GPS pose des problèmes à l'intérieur des bâtiments ou en milieu urbain dense en raison d'une mauvaise communication avec les satellites. Or la localisation présente un intérêt certain à l'intérieur des bâtiments par exemple pour le suivi des personnes âgées à leur domicile, de pompiers lors de leurs interventions, ou de visiteurs d'une exposition. La localisation par capteur inertiel (utilisant un accéléromètre et/ou un gyromètre et/ou un magnétomètre) peut alors être une alternative ou un complément intéressant au GPS, puisque c'est un système autonome toujours disponible et qu'il est en outre moins cher que le GPS.

L'estimation des orientations peut également être utilisée, par exemple, pour l'analyse de la marche ou de la course, pour des applications médicales, de jeux, ou d'entraînement sportif. L'estimation de ces orientations est d'autant plus intéressante que les orientations sont estimées pour l'ensemble des segments de la chaîne articulée. On peut ainsi représenter le mouvement étudié dans son ensemble (par exemple, pour animer un avatar, étudier l'angle du genou ou de la hanche).

Il est connu des systèmes de capture de mouvement qui s'avèrent être généralement coûteux ou incapables d'estimer à la fois la position et l'orientation de l'ensemble des segments de la chaîne articulée utilisés pour la locomotion.

Les systèmes de capture de mouvement généralement utilisés, par exemple pour le cinéma, comprennent des dispositifs optiques (systèmes commercialisés par Vicon, ou Codamotion), magnétiques (systèmes commercialisés par Polhemus). Ces dispositifs sont capables d'estimer le mouvement (position et orientation) du corps entier mais ils sont de coût élevé. De plus ils nécessitent d'équiper l'environnement dans lequel s'effectue le déplacement, et ont une zone de couverture réduite.

Récemment, des systèmes de capture de mouvement à base de microsystèmes électromécaniques ou MEMS pour "Micro-Electro-Mechanical System" en langue anglaise, ont été développés. Ces dispositifs appelés centrales d'attitude (commercialisés par exemple par Movea, Xsens, Microstrain) sont généralement composés d'un magnétomètre tri-axes, d'un accéléromètre tri-axes et d'un gyromètre tri-axes ou d'un sous ensemble de ces trois capteurs de mouvement. Ils permettent d'estimer l'orientation du corps rigide mobile auquel ils sont fixés, mais pas la position. Dans le cas d'une chaîne articulée, il est nécessaire d'utiliser un capteur par segment de la chaîne articulée pour laquelle on cherche à estimer l'orientation. La mise en place du système s'avère alors contraignante pour l'utilisateur. Par contre, la zone de couverture n'est pas réduite.

Des systèmes ont été proposés pour fusionner les données de plusieurs capteurs inertiels disposées sur le corps avec un modèle biomécanique et/ou de chaîne articulée.

La demande de brevet américain US 2008/0285805 propose une méthode de capture de mouvement d'un objet possédant au moins deux segments équipés chacun d'au moins un capteur de mouvement. Les mesures des capteurs de mouvement sont collectées, chaque capteur de mouvement comprenant au moins un accéléromètre triaxial et un gyromètre triaxial (3A3G). L'orientation et la position de chaque segment est calculée en intégrant les mesures délivrées par les gyromètres et en intégrant deux fois les mesures délivrées par les accéléromètres en tenant compte dans les calculs de contraintes sur les segments.

Leur solution est générale, et ne tient pas compte de la spécificité du type d'activité qui met en oeuvre des mouvements complémentaires de plusieurs membres. Par exemple, dans le cas de la marche, les mouvements des deux membres sont similaires et décalés dans le temps et les degrés de liberté des deux membres sont comparables. En outre, il est uniquement décrit des modes de réalisations utilisant systématiquement un capteur au moins 3A3G par membre, ce qui est une réalisation présentant un nombre élevé de capteurs, de coût élevé.

La demande de brevet EP1984696, propose un système d'estimation du mouvement d'une chaîne articulée constituée de N segments consécutifs comprenant des moyens de mesure de l'accélération d'un segment de la chaîne et des moyens de mesure de l'orientation sur chacun des segments. La présence d'un capteur par segment étant nécessaire, le système est de coût élevé.

La demande de brevet EP 1 990 138 A1, porte sur un procédé d'estimation du mouvement d'une chaîne articulée constituée de plusieurs segments. Chaque segment est équipé d'un capteur et utilise des modèles de mesures et un observateur. Ainsi, un nombre important de capteurs est nécessaire, et le coût et l'encombrement est élevé. De plus, la complexité algorithmique est relativement élevée.

Pour étudier la marche ou la navigation, il est connu des dispositifs utilisant des capteurs inertiels disposés sur les membres inférieurs, dans lesquels des capteurs sont généralement disposés sur le pied ou sur le tibia et parfois sur la cuisse.

Pour la navigation, il est possible de regrouper les systèmes en deux catégories. Une première catégorie de systèmes utilise des relations entre des paramètres extraits des mesures comme la variance de l'accélération ou la longueur des pas, comme par exemple décrit dans la demande de brevet américain US 7467060. Ces relations sont de précision limitée et varient d'un utilisateur à un autre.

Une deuxième catégorie de systèmes mettent en oeuvre une double intégration de l'accélération propre. Ces systèmes nécessitent d'utiliser des accéléromètres. Afin d'obtenir l'accélération propre, il est nécessaire de compenser la gravité des mesures accélérométriques et donc de connaître l'orientation à tout instant. Ces systèmes nécessitent donc également soit un capteur permettant d'estimer l'angle tel un gyromètre ou un magnétomètre (WO 2010/046364), soit un modèle d'évolution angulaire (US2002/0002863).

Ces systèmes n'utilisent pas d'information sur la chaîne articulée et le rapport performance/coût est faible.

Ces différents systèmes ont une précision limitée et un coût élevé.

Il est proposé un système selon les revendications 6 à 10. Un tel système permet à coût réduit d'avoir l'attitude de chacun des segments de la chaîne articulée et le déplacement de n'importe quel point particulier de cette chaîne

Dans un mode de réalisation, lesdits moyens de mesure comprennent un magnétomètre à au moins un axe de mesure et/ou un gyromètre à au moins un axe de mesure.

Ainsi l'encombrement et le coût sont limités.

Selon un mode de réalisation, lesdits premiers moyens de détermination comprennent un magnétomètre à au moins un axe de mesure et/ou un gyromètre à au moins un axe de mesure fixé sur chaque dit autre membre.

Ainsi, l'orientation de chacun des segments peut être obtenue avec une précision améliorée. D'autre part, le système s'adapte plus rapidement aux changements de vitesse.

Dans le mode de réalisation, lesdits premiers moyens de détermination sont adaptés pour calculer la valeur du ou desdits deuxièmes paramètres à partir de la valeur dudit premier paramètre par un modèle d'évolution temporelle liant les valeurs dudit premier paramètre à celles du ou desdits deuxièmes paramètres.

Ainsi, le nombre de capteurs est minimisé (il n'est pas nécessaire d'instrumenter tous les membres de la chaîne articulée).

Selon un mode de réalisation, ledit modèle d'évolution comprend un décalage temporel entre la valeur dudit premier paramètre et la valeur du ou desdits deuxièmes paramètres.

Ainsi, le modèle d'évolution est simplifié et prend en compte une caractéristique communément rencontrée dans le type de mouvement traité.

Dans un mode de réalisation, ledit modèle d'évolution est adapté pour lier l'orientation de tous les segments de ladite chaîne articulée à celle dudit premier segment.

Ainsi, l'instrumentation d'un seul segment de la chaîne articulée permet de remonter à l'orientation de l'ensemble des segments de la chaîne articulée.

Selon un mode de réalisation, lesdits deuxièmes moyens de détermination comprennent un accéléromètre à au moins deux axes de mesure.

Ainsi, la précision peut être améliorée.

Dans un mode de réalisation, ladite chaîne articulée représente deux membres supérieurs d'un être vivant ou artificiel.

Selon un mode de réalisation, ladite chaîne articulée représente deux membres inférieurs d'un être vivant ou artificiel.

Dans un mode de réalisation, ladite chaîne articulée comprend deux segments par membre inférieur, et six degrés de liberté.

Ainsi, l'estimation de la pose des membres inférieurs tout au long d'un déplacement de l'être vivant ou artificiel peut être effectuée avec un nombre de degré de liberté et un nombre de capteurs minimisé.

Selon un mode de réalisation, ladite chaîne articulée comprend trois segments par membre inférieur, et huit degrés de liberté.

Ainsi, on peut mettre le capteur au pied (cas le moins contraignant pour l'utilisateur et économiquement le plus intéressant car le capteur peut être dans la chaussure). Si on connaît les modèles d'évolution temporelle liant tous les angles, on peut remonter à tous les angles de la chaîne articulée.

Dans un mode de réalisation lesdits moyens de mesure, lesdits premiers moyens de détermination, et lesdits deuxièmes moyens de détermination, sont adaptés pour être fixé sur la chaîne articulée à un endroit correspondant à un tibia.

Ainsi les phases de pose du pied sur le sol peuvent être facilement déterminées (capteurs angulaires au niveau du tibia).

Dans un mode de réalisation, lesdits moyens de mesure, lesdits premiers moyens de détermination, et lesdits deuxièmes moyens de détermination, sont adaptés pour être fixés sur la chaîne articulée à un endroit correspondant au pied ou à la cheville.

Il est ainsi possible d'exploiter l'information de vitesse nulle pour limiter la dérive due à l'intégration utilisée pour estimer la position.

Selon un mode de réalisation, le système comprend des premiers moyens de détection de pose sur le sol d'un pied de la chaîne articulée.

Ainsi, les phases du pied à vitesse nulle sont facilement déterminées.

En outre, le système peut comprendre des moyens d'estimation de la distance inter-pieds.

Ainsi, le système nécessite l'instrumentation d'un seul membre, et la précision de la trajectoire des pieds peut être améliorée.

Dans un mode de réalisation, le système comprend des deuxièmes moyens de détection de cycles de déplacement ou d'instants particuliers caractéristiques de ces cycles.

Ainsi, on peut ajuster les paramètres des modèles d'évolution temporelle et en déduire le mouvement sur la durée du cycle. Il est également proposé un procédé selon les revendications 1 à 5. Le modèle d'évolution temporelle comportant une relation de dépendance temporelle, notamment une relation de décalage temporel, entre les mouvements d'au moins deux membres distincts de l'entité, ledit au moins un autre paramètre comporte un paramètre représentatif d'un mouvement d'un autre segment de la chaîne articulée associé à au moins une partie d'un autre membre de la chaîne articulée que le membre représenté au moins par le premier segment.

De façon optionnelle également, le modèle d'évolution temporelle comporte une relation de dépendance temporelle des orientations de tous les segments de la chaîne articulée relativement à celle du premier segment.

De façon optionnelle également, l'entité est représentée sous la forme d'une chaîne articulée représentant deux membres supérieurs ou inférieurs d'un être vivant ou artificiel :

- dans laquelle chaque membre est lui-même représenté par deux segments reliés entre eux par une articulation, la chaîne articulée présentant six degrés de liberté, ou
- dans laquelle chaque membre est lui-même représenté par trois segments reliés entre eux deux à deux par deux articulations, la chaîne articulée présentant huit degrés de liberté.

L'invention a également pour objet un programme d'ordinateur téléchargeable depuis un réseau de communication et/ou enregistré sur un support lisible par ordinateur et/ou exécutable par un processeur, caractérisé en ce qu'il comprend des instructions pour l'exécution des étapes d'un procédé de détermination selon l'invention, lorsque ledit programme est exécuté sur un ordinateur.

De façon optionnelle également, un système de détermination selon l'invention peut comporter en outre au moins l'un des éléments de l'ensemble constitué de premiers moyens de détection de pose sur le sol d'un pied de l'entité représentée par ladite chaîne articulée, de moyens d'estimation d'une distance inter-pieds et de seconds moyens de détection de cycles de déplacement ou d'instants particuliers caractéristiques de ces cycles.

L'invention sera mieux comprise à l'étude de quelques modes de réalisation décrits à titre d'exemples nullement limitatifs et illustrés par les dessins annexés sur lesquels :

- la figure 1 illustre un mode de réalisation d'un système selon un aspect de l'invention ;
- la figure 2 illustre un exemple de solution géométrique ;
- la figure 3 représente les repères global fixe NF et local BF ;
- les figures 5a, 5b et 5c illustrent la trajectoire d'une cheville d'un piéton lors d'un cycle de marche ;
- la figure 6 illustre la vitesse angulaire du pied droit du piéton de la trajectoire des figures 5a, 5b et 5c ;
- les figures 7a et 7b illustrent respectivement l'accélération instantanée du pied du piéton calculée à partir de la trajectoire du pied et la mesure d'un accéléromètre exprimée dans le repère NF simulée à partir de cette accélération ;
- les figures 8a et 8b illustrent les accélérations subies par la cheville du piéton durant un cycle de marche des figures 5a, 5b et 5c montrant la norme et la direction de l'accélération, trois vecteurs d'accélération à trois instants différents sont représentés ;
- les figures 9a, 9b, 9c et 9d représentent le principe de fonctionnement d'un accéléromètre à deux axes de mesure ayant une masse sismique fixée à des ressorts ;

- les figures 10a et 10b illustrent une détection de pas lors de la marche d'un piéton à partir de la norme de l'accélération mesurée par un capteur fixé à la cheville d'un piéton, avec une fréquence d'échantillonnage de 200 Hz ;
- les figures 12a, 12b, 12c, 12d et 12e représentent les valeurs extrema des angles d'une jambe au cours d'un cycle de marche à pied ;
- les figures 13a et 13b représentent une simulation des angles durant une marche ;
- les figures 14a, 14b, 14c, 14d, 14e, et 14f représentent les extrema des angles des jambes durant une marche pour différentes pentes ; et
- les figures 15a, 15b, 16a, et 16b illustrent le fonctionnement d'un système selon un aspect de l'invention comprenant deux magnétomètres triaxiaux montés sur les chevilles d'un piéton.

Dans l'ensemble des figures, les éléments ayants les mêmes références sont similaires.

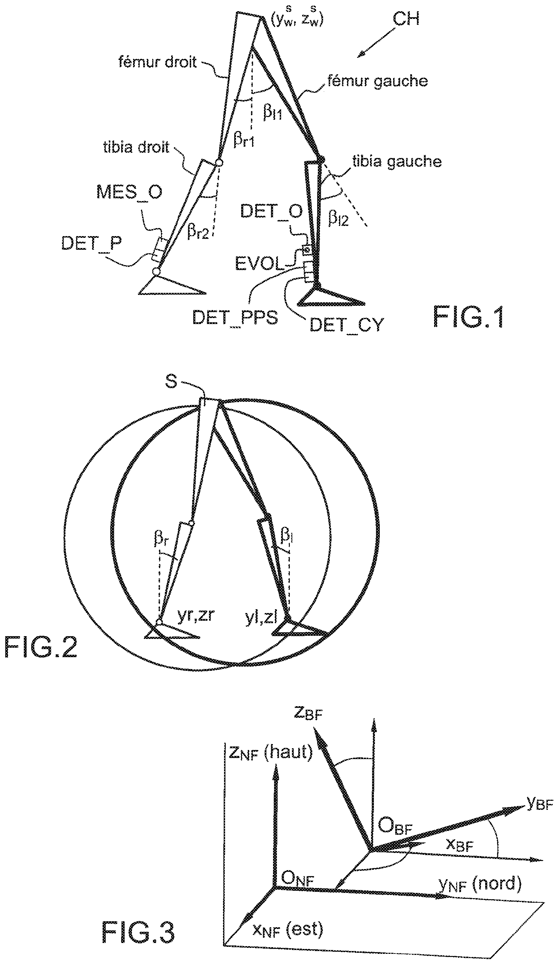

Sur la figure 1, est représenté un système de détermination de valeurs de paramètres représentatifs d'un mouvement d'au moins deux membres d'une entité pour engendrer un déplacement de ladite entité. L'entité est adaptée pour être représentée sous la forme d'une chaîne articulée CH, comprenant au moins deux segments reliés par une articulation. En l'occurrence, la chaîne articulée de la figure 1 comprend cinq articulations et six segments. Les six segments sont les deux cuisses, les deux tibias et les deux pieds, et les cinq articulations sont la hanche, les deux genoux et les deux chevilles. Bien entendu, cet exemple de chaîne articulée n'est pas limitatif. Les articulations de genoux, de hanche ou de chevilles sont considérées à un degré de liberté. On considère le mouvement dans un plan orthogonal aux axes de rotations des articulations, ces axes étant tous colinéaires. Ainsi, la chaîne articulée comprend huit degrés de liberté, à savoir l'angle des différents segments avec la verticale et la position cartésienne en deux dimensions d'un point particulier de la chaîne articulée, par exemple la taille.

Si on considère, en variante, que la chaîne articulée ne comprend pas les pieds, et donc pas les chevilles, mais que les conditions de mouvement sont identiques (dans un plan orthogonal aux axes de rotation des différentes articulations, ces différents axes étant tous colinéaires), la chaîne articulée comprend alors quatre segments, trois articulations et six degrés de liberté.

Durant une plage temporelle donnée un point de la chaîne articulée CH est considéré comme fixe. Par exemple, pendant une partie du cycle de marche, le pied est posé au sol. Ainsi la position ne dépend plus que des angles articulaires et de la longueur des segments, et le nombre de degrés de liberté est diminué.

En outre, les angles articulaires sont bornés, i.e. compris entre une valeur minimale et une valeur maximale, les articulations du corps humain ayant des butées articulaires.

Le système comprend un module de mesure MES_O de la valeur d'un premier paramètre $\beta_r$ représentatif d'une orientation d'un premier segment d'un premier membre, en l'espèce la jambe droite. Le système comprend, en outre, un premier module de détermination DET_O de la valeur d'un deuxième paramètre $\beta_l$ représentatif d'une orientation d'un deuxième segment appartenant à l'autre membre de la chaîne articulée CH, la jambe gauche. Lorsque la chaîne articulée comprend plus de deux membres, chaque autre membre, différent du premier membre muni du module de mesure MES_O, est muni d'un module respectif de détermination DET_O de la valeur d'un deuxième paramètre représentatif d'une orientation d'un deuxième segment appartenant à cet autre membre.

Le système comprend également un deuxième module de détermination DET_P de la valeur d'un troisième paramètre représentatif de l'évolution temporelle d'une position cartésienne P d'un point en mouvement lié à la chaîne articulée CH. Le troisième paramètre représentatif de l'évolution temporelle d'une position P d'un point en mouvement lié à la chaîne articulée CH, peut être la position cartésienne, la vitesse ou l'accélération du point P.

Le module de mesure METS_O peut comprendre un magnétomètre à au moins un axe de mesure et/ou un gyromètre à au moins un axe de mesure. Le premier module de détermination DET_O peut comprendre un magnétomètre à au moins un axe de mesure et/ou un gyromètre à au moins un axe de mesure fixé sur un segment de l'autre membre.

Le premier module de détermination DET_O est adapté pour déterminer, par un capteur et/ou un modèle d'évolution, la valeur du ou desdits deuxièmes paramètres $\beta_l$ à partir de la valeur du premier paramètre $\beta_r$ par un modèle EVOL d'évolution temporelle liant les valeurs du premier paramètre $\beta_r$ aux valeurs du ou desdits deuxièmes paramètres $\beta_l$.

Le modèle EVOL peut comprendre un décalage temporel entre la valeur du premier paramètre $\beta_r$ et la valeur du ou desdits deuxièmes paramètres $\beta_l$.

En variante, Le modèle EVOL peut être adapté pour lier l'orientation de tous les segments de ladite chaîne articulée à celle dudit premier segment. Le deuxième module de détermination DET_P comprend, par exemple, un accéléromètre à au moins deux axes de mesure.

En variante, la chaîne articulée CH peut représenter deux membres supérieurs d'un être vivant ou artificiel, notamment lorsque le déplacement de l'entité est, par exemple, un déplacement par actionnement ou simulation d'actionnement de rames, d'aviron ou de pagaies, sur un canoë, un kayak, ou un rameur. Dans ce cas, les segments cuisse, tibia et pied sont respectivement remplacés par les segments bras, avant-bras, main.

L'accéléromètre peut être adapté pour être fixé sur la chaîne articulée CH à un endroit correspondant à une cheville sur un tibia.

Le système peut également comprendre des premiers moyens de détection DET_PPS de pose sur le sol d'un pied de la chaîne articulée CH. Il est possible alors de prendre la position $P_r$ égale à $P_0$ pendant toute la durée du pas, $P_0$ étant la dernière position estimée avant la pose du pied. La position $P_l$ de l'autre pied peut être estimée à l'aide d'un module d'estimation EST_DIP apte à restituer la distance inter-pieds, par exemple par un dispositif télémétrique radio. La distance inter-pied, associée au modèle d'évolution, permet de savoir où l'on se trouve dans le cycle de marche pour le deuxième pied. D'autre part, on a une information supplémentaire sur la longueur de la foulée lorsque le pied est posé, ce qui améliore la précision du système. L'utilisation d'un système de mesure de distance n'est qu'un exemple d'utilisation de la détection de la pose de pied mais cette dernière est très utile en dehors de ce mode de réalisation particulier.

Le système peut comprendre des deuxièmes moyens de détection DET_CY de cycles de déplacement. Un cycle pour la marche correspond à la succession d'un pas de chaque pied. Ce cycle correspond à la pseudo-période entre l'occurrence de deux événements similaires : entre deux instants auxquels le talon heurte le sol HS ou entre deux décollages des orteils TO ou entre 2 instants où le pied est à plat FF. Pour obtenir la position et l'orientation d'un point de la chaîne articulée, plusieurs moyens sont envisageables.

Les positions sont décrites par trois coordonnées cartésiennes dans un repère lié au repère terrestre.

Les orientations peuvent être décrites par trois angles ou par un quaternion décrivant les rotations permettant de passer d'un repère lié au repère terrestre à un repère lié au membre pour lequel on veut mesurer l'orientation.

On entend par paramètres représentatifs de la position et de l'orientation, tout ou partie des variables exprimant la position ou l'orientation. Le choix de ces variables dépend du nombre de degrés de libertés de la chaîne articulée CH.

Par exemple, dans le cas de la marche en avant ou en arrière on a besoin de seulement trois informations : les deux coordonnées dans le plan de la marche et l'angle de rotation autour de l'axe médio-latéral.

Un mode de réalisation consiste à positionner un capteur inertiel par membre.

Un capteur inertiel, compris dans les moyens de mesure MES_O, doit être apte à mesurer un paramètre représentatif de l'orientation et de la position de l'élément auquel il est lié. Ainsi, un accéléromètre à au moins deux axes de mesure sert à la mesure de la position et un gyromètre à au moins un axe de mesure ou un magnétomètre à au moins un axe de mesure sert à la mesure de l'orientation.

Préférentiellement, un capteur inertiel est situé sur le tibia au niveau de la cheville de chaque jambe. Ainsi, on évite d'avoir à estimer l'angle du pied. En outre, la proximité avec le pied permet de faire l'hypothèse d'une vitesse nulle lorsque le pied est posé, et d'éviter ainsi la dérive lors de l'intégration de l'accélération propre issue de l'accéléromètre.

Le calcul de la trajectoire de chaque centrale, mis en oeuvre dans le deuxième module de détermination DET_P, passe par la détection des pas, réalisés par le module de détection de cycles DET_CY puis le module de détection DET_PPS, puis le calcul de l'orientation à chaque instant de mesure pendant la durée du pas. Cette orientation est utilisée pour exprimer la mesure de l'accéléromètre dans un repère lié au repère terrestre. Ceci permet de compenser la gravité et d'utiliser l'hypothèse de vitesse nulle en début et en fin de pas. L'accélération propre ainsi obtenue est ensuite intégrée deux fois. On prendra par exemple, pour réaliser le deuxième module de détermination DET_P, le système décrit dans la demande de brevet WO2010046364.

Une autre solution permet de minimiser le nombre de capteurs. Par exemple, la trajectoire, déterminée par le deuxième module de détermination DET_P, et l'orientation, déterminée par le module de mesure MES_O, d'un point d'un premier membre est obtenu par une première centrale. La trajectoire d'un point du deuxième membre et/ou l'orientation du deuxième membre est obtenue en décalant temporellement le signal de la première centrale (signaux bruts des capteurs ou traités de position et d'orientation obtenus après traitement). En effet, il existe souvent dans la locomotion une relation temporelle entre le mouvement des différents membres.

Par exemple, dans le cas de la marche, le mouvement des jambes est décalé d'un demi cycle de marche. Dans le cas de l'aviron le mouvement des deux bras est symétrique.

Ensuite, on calcule des paramètres de la chaîne articulée à partir des trajectoires et des angles.

Etant donné le modèle de chaîne articulée, il est possible d'écrire les informations précédemment calculées (angles par les modules MES_O et DET_O, et positions par le module DET_P) comme des fonctions des paramètres recherchés (angles articulaires, position globale de la chaîne CH). Les valeurs des paramètres de la chaîne articulée CH sont ceux qui permettent de vérifier au mieux les informations précédemment calculées (angles et positions). Ces valeurs peuvent être obtenues par toute méthode d'inversion de modèle (optimisation, filtrage de Kalman, géométrie...).

Dans un mode de réalisation, on utilise les notations suivantes:

Les coordonnées sont donnés dans le repère (Y, Z), Y étant l'axe donné par la direction de la marche et Z étant l'axe vertical vers le haut.

$(y_w, z_w)$ : Coordonnées de la hanche
$(y_{al}, z_{al})$ : Coordonnées de la cheville gauche
$(y_{ar}, z_{ar})$ : Coordonnées de la cheville droite
$\beta_{11}$ : angle de la cuisse gauche par rapport à la verticale

$\beta_{l2}$ : angle du tibia gauche par rapport à la cuisse gauche

$\beta_{l}$ : angle du tibia gauche par rapport à la verticale

$\beta_{r1}$ : angle de la cuisse droite par rapport à la verticale

$\beta_{r2}$ : angle du tibia droit par rapport à la cuisse droite

$\beta_{r}$ : angle du tibia droit par rapport à la verticale

$L_{l1}$ : longueur de la cuisse gauche

$L_{l2}$ : longueur du tibia gauche

$L_{r1}$ : longueur de la cuisse droite

$L_{r2}$ : longueur du tibia droit

Décrire la cinématique du corps humain est très difficile. Par exemple, une jambe a 29 os et 37 muscles. Cependant, à partir de l'analyse de la marche de piétons, on modélise la partie des jambes du piéton située entre la taille (les hanches) et la cheville.

Comme illustré sur la figure 1, lors d'une marche, les jambes du piéton sont représentées par quatre segments liés par trois articulations. Le fémur et le tibia sont représentés par deux segments rigides reliés par une articulation représentant le genou, à un axe de rotation (un degré de liberté). Les deux jambes du piéton sont connectées au niveau du fémur avec la taille, avec une connexion permettant trois degrés de liberté de rotation du fémur.

On ne s'intéresse pas à une marche de côté ou à une marche en arrière, mais à une marche en avant. Par conséquent, on suppose que le fémur a un seul degré de liberté dans le plan sagittal défini par les axes $(y_{SF}, z_{SF})$. Par conséquent, la taille est représentée par une seule articulation à laquelle les deux jambes sont connectés permettant deux angles de rotation $\beta_{r1}$ et $\beta_{l1}$.

Aussi, le modèle biomécanique considéré a six degrés de liberté, et peut être représenté par quatre angles $\beta_{r1}, \beta_{r2}, \beta_{l1}, \beta_{l2}$ et les coordonnées cartésiennes à deux dimensions $(y_w^s, z_w^s)$ de la taille du piéton.

$\beta_r$ et $\beta_l$ désignent respectivement les angles des tibias droit et gauche par rapport à la direction verticale descendante selon les relations suivantes :

$$\begin{aligned} \beta_r &= \beta_{r1} + \beta_{r2} \\ \beta_l &= \beta_{l1} + \beta_{l2} \end{aligned} \qquad\qquad (1a)$$

Lors d'une marche les angles des jambes varient dans un intervalle limité et ont une forme similaire pour chaque pas. Les angles de genoux $\beta_{r2}$ et $\beta_{l2}$ doivent toujours être négatifs.

Dans la suite, $\beta$, $\beta_1$, et $\beta_2$ sont utilisés, pour les deux jambes, pour désigner respectivement $\{\beta_r, \beta_l\}$, $\{\beta_{r1}, \beta_{l1}\}$, et $\{\beta_{r2}, \beta_{l2}\}$.

Dans la suite de ce mode de réalisation, il est possible de procéder de deux manières :

- soit, pour chaque échantillon temporel, on réalise l'ensemble des étapes qui suivent et on itère avec l'échantillon suivant ;
- soit, on réalise chacune des étapes sur l'ensemble des échantillons temporels pendant la durée du pas avant de passer à l'étape suivante.

La première solution est celle qui aboutit aux meilleures performances.

On effectue des mesures avec un accéléromètre et un magnétomètre sur chaque tibia au niveau de la cheville.

Dans une variante, les mesures effectuées sur le deuxième tibia peuvent être obtenues en faisant un décalage temporel d'un demi cycle de marche des mesures du premier tibia.

On calcule la trajectoire de chacun des deux pieds et les angles $\beta_l$, $\beta_r$ des tibias par rapport à la verticale à chaque instant comme par exemple décrit dans le document WO 2010/046364. On obtient ainsi six grandeurs (quatre positions et deux orientations) G = $\{y_{al}, z_{al}, \beta_l, y_{ar}, z_{ar}, \beta_r\}$.

Ce qui précède peut, en variante, être effectué par un accéléromètre et un gyromètre, ou par un accéléromètre, un magnétomètre et un gyromètre fixés à la cheville, et une méthode d'estimation de la trajectoire connue.

L'ensemble des paramètres représentant le mouvement de la chaîne articulée CH peut être décrit par un ensemble PAR de paramètres. Sur la figure 1, les paramètres à déterminer sont $PAR=\{y_w, z_w, \beta_{l1}, \beta_{l2}, \beta_{r1}, \beta_{r2}\}$. Les paramètres connus sont l'ensemble L des longueurs des tibias et des cuisses : $L=\{L_{r1}, L_{r2}, L_{l1}, L_{l2}\}$.

On cherche un ensemble PAR* de paramètres qui vérifient au mieux les informations G. On peut les obtenir en cherchant les paramètres qui minimisent une distance, au sens mathématique, entre G et f(PAR*,L) avec G=f(P,L) ; f représentant la fonction qui lie les informations G estimées précédemment aux paramètres connus de la chaîne articulée CH et aux paramètres liés au mouvement de cette chaîne articulée CH.

Cette minimisation peut, par exemple, être faite par un algorithme d'optimisation tel que l'algorithme de Levenberg-Marquard.

La fonction f est donnée par le système d'équations suivant :

$$\begin{cases} y_{ra} = y_w + L_{r1}\sin(\beta_{r1}) + L_{r2}\sin(\beta_{r1} + \beta_{r2}) \\ z_{ra} = z_w - L_{r1}\cos(\beta_{r1}) - L_{r2}\cos(\beta_{r1} + \beta_{r2}) \\ \beta_r = \beta_{r1} + \beta_{r2} \\ y_{la} = y_w + L_{l1}\sin(\beta_{l1}) + L_{l2}\sin(\beta_{l1} + \beta_{l2}) \\ z_{la} = z_w - L_{l1}\cos(\beta_{l1}) - L_{l2}\cos(\beta_{l1} + \beta_{l2}) \\ \beta_l = \beta_{l1} + \beta_{l2} \end{cases} \qquad (2a)$$

Il est possible d'ajouter des contraintes. Une contrainte peut être que l'angle du genou est toujours positif : $\beta_{l2}$ > 0 et/ou $\beta_{r2}$ >0>0. Une autre contrainte peut être que lorsqu'un pied est posé (gauche ou droit), ses coordonnées ($y_{la}$ et $z_{la}$ pour le pied gauche posé et $y_{ra}$ et $z_{ra}$ pour le pied droit posé) sont constantes et égales aux coordonnées estimées lors du premier instant du posé de pied.

Il est également possible de dériver les équations précédentes et travailler sur les vitesses.

Une solution géométrique peut être obtenue comme illustré sur la figure 2.

Pour chaque jambe, étant donnés la position de la cheville ($y_a$,$z_a$), l'angle β et la longueur du tibia $L_2$, il est possible de déterminer la position du genou. Pour cela on trace un segment représentant le tibia ayant une première extrémité de coordonnées ($y_a$,$z_a$), une longueur $L_2$ et une orientation β par rapport à la verticale. Les coordonnées du genou sont alors données par les coordonnées de l'autre extrémité du segment. Ainsi, il est ensuite possible de tracer un cercle ayant pour centre la position du genou et pour rayon la longueur de la cuisse. La hanche se trouve à l'intersection des deux cercles tracés pour les deux jambes. La hanche ne peut pas se trouver sous le sol. Il y a donc une seule solution S. Une fois la position S de la hanche déterminée, les segments des cuisses peuvent être tracés et les angles articulaires déduits.

Ci suit un autre mode de réalisation de l'invention, pour une chaîne articulée CH représentant deux membres inférieurs d'un être vivant ou artificiel.

Un capteur, du module de mesure MES_O, est utilisé pour permettre d'estimer un paramètre représentatif de l'angle d'au moins un des segments de la chaîne articulée CH.

On utilise un capteur d'angle ou de vitesse angulaire, par exemple un magnétomètre ou un gyromètre à au moins un axe de mesure. L'estimation de l'angle ou de la vitesse angulaire à partir d'un magnétomètre ou d'un gyromètre est connue et est décrite par la suite.

Dans un mode avantageux ce capteur est positionné sur un tibia. En effet, sur un tibia, la courbe d'évolution des angles est plus riche, i.e., elle présente plus de points singuliers utiles pour les comparaisons décrites dans la suite de la description.

Dans un mode avantageux, un capteur est positionné sur chaque membre. Ce mode de réalisation permet de réaliser la fusion de deux capteurs afin d'améliorer la précision du système. Il permet, entre autres, de prendre en compte les changements de vitesse de manière plus précise.

Dans un mode avantageux, on utilise un modèle EVOL d'évolution temporelle de l'angle d'au moins un segment de la chaîne articulée CH. Ainsi, au cours de temps, les angles ne peuvent pas évoluer d'une manière quelconque mais suivent une loi d'évolution qui tient compte des mouvements effectués durant le mouvement. Ce modèle d'évolution EVOL est caractérisé par des minima et des maxima.

Un exemple de modèle d'évolution peut être une combinaison linéaire ou non de fonctions trigonométriques du temps (telles des cosinus et des sinus) dont la fréquence et l'amplitude sont des paramètres à déterminer. De manière avantageuse, l'angle entre la cuisse et la verticale est défini par un minimum local et un maximum local durant chaque cycle de mouvement d'un mouvement sensiblement cyclique, par exemple un mouvement de marche.

L'angle entre la cuisse et le tibia est défini par deux minima locaux et deux maxima locaux définissant les différentes phases du cycle de marche. Les courbes d'évolution temporelle EVOL des différents angles de la chaîne articulée CH sont liées entre-elles. Ainsi, la détermination d'un angle d'un segment de la chaîne CH permet de déduire les angles qui sont liés à lui. Ceci, diminue fortement le nombre de capteurs nécessaires.

On peut comparer l'angle estimé avec l'angle du modèle d'évolution EVOL, pour déterminer à quel endroit du cycle de marche on se situe, ainsi que les paramètres du modèle d'évolution EVOL d'angles précédemment décrit.

Un exemple avantageux de comparaison consiste à détecter des mimima et/ou des maxima locaux et/ou des passages par zéro de la fonction d'angle ou de vitesse angulaire. On peut ainsi déterminer les différentes phases de la marche ainsi que la valeur des angles à des instants particuliers de la marche: maintien du pied à plat ou FF pour "Foot Flat" en langue anglaise correspondant au maintien du bassin au dessus du pied; pose du pied au sol ou HS pour "Heel Strike" en langue anglaise correspondant au début de la phase d'amortissement qui s'achève lorsque le bassin passe au dessus du pied; instant où le pied va quitter le sol ou TO pour "Toe Off" en langue anglaise; instant auquel le pied repart vers l'avant ou MS pour "Mid Swing" correspondant au milieu de la phase de vol du pied; instant de vitesse angulaire nulle ou ZC pour "zéro crossing" quand la vitesse angulaire passe par zéro.

Grâce au modèle d'angles EVOL, et aux valeurs des angles en ces points, il est possible d'ajuster les paramètres des modèles et d'en déduire le mouvement complet de la chaîne articulée CH entre les points.

Parmi les paramètres, on peut déterminer l'inclinaison du sol sur lequel se déplace l'utilisateur, ce qui est très utile pour détecter les montées et les descentes.

Par exemple, on définit tout d'abord les repères ou "frames" en langue anglaise, puis on détermine les modèles des mesures exploitées. Les phases de la marche sont ensuite rappelées et une manière classique de détection des pas est donnée.

Les vecteurs sont mesurés dans un référentiel lié au capteur. Ainsi, les mesures dépendent de l'orientation du capteur. Comme les capteurs sont attachés à un corps en mouvement, les mesures sont sensibles aux mouvements instantanés. Cela nécessite des mesures instantanées de rotation, car l'attitude ou orientation du capteur change au cours du temps.

On définit trois repères pour clarifier les transformations à effectuer dans la suite de la description. Le repère Nord ou NF "North Frame" en langue anglaise, le repère local ou BF pour "Body Frame" en langue anglaise, et le repère intermédiaire ou SF pour "Step Frame" en langue anglaise, sont respectivement définis par les axes $(x_{NF}, y_{NF}, z_{NF})$, $(x_{BF}, y_{BF}, z_{BF})$, $(x_{SF}, y_{SF}, z_{SF})$ et les origines $O_{NF}, O_{BF}, O_{SF}$.

Le repère Nord NF est un repère fixe par rapport au référentiel terrestre durant l'ensemble du mouvement, par exemple de marche ou de course. Le repère Nord NF peut être considéré comme le repère de référence : les orientations ou positions angulaires et les positions ou positions cartésiennes sont mesurées dans ce repère. Les axes se réfèrent au repère terrestre par les directions Est, Nord, et Haut. Son origine $O_{NF}$ peut correspondre à l'endroit où le mouvement débute ou à tout autre endroit. Les données exprimées dans ce repère sont notées avec un exposant n.

Le repère local ou lié au capteur BF est le référentiel de dans lequel les données brutes sont mesurées. Son centre est $O_{BF}$. Les données dans ce repère sont notées avec un exposant b. Ses axes $(x_{BF}, y_{BF}, z_{BF})$ correspondent aux axes des capteurs (dans le cas de capteurs triaxiaux) à chaque instant. Les coordonnées de son origine $O_{BF}$ dans le repère global fixe NF correspondent à l'emplacement du capteur dans le repère global fixe NF à chaque instant.

Le repère intermédiaire SF est un repère lié à la Terre, mais seulement pendant un intervalle de temps $[t_1;t_2]$ (par exemple au cours d'une étape). Ce repère est utilisé avec un capteur de mouvement avec moins de six degrés de liberté ou 6 DOF pour "Degree Of Freedom" en langue anglaise. Par exemple, il est utilisé quand l'ensemble capteur de mouvement EC a un axe de rotation invariant et/ou un mouvement plan durant l'intervalle de temps $[t_1;t_2]$. $x_{SF}$ est l'axe de rotation et l'ensemble capteur EC est soumis à un mouvement plan dans le plan défini par les axes $(y_{SF}, z_{SF})$. Chaque fois que le plan est vertical $y_{SF}$, est choisi pour être l'axe horizontal, tandis que l'axe $z_{SF}$ est l'axe vertical. Son origine correspond à la place du capteur au début intervalle de temps (par exemple à t=t1). Les données exprimées dans ce repère sont notées avec un exposant s.

La figure 3 représente les repères global fixe NF et local BF. Comme on le voit, le repère local BF est lié au repère global fixe NF par une translation et une rotation. Toutes les données inertielles sont mesurées dans le repère local BF. Le repère local BF subit une translation et une rotation par rapport au repère global fixe NF.

Le repère intermédiaire SF est lié au repère global fixe NF par une translation invariante dans le temps et une rotation invariante dans le temps pour l'ensemble de l'intervalle de temps $[t_1;t_2]$. Ici, le repère local BF tourne autour de l'axe de rotation $x_{SF}$ et dans certains cas, il est supposé que la translation de $O_{BF}$, pour $t \in [t_1,t_2]$ est dans le plan défini par les axes $(y_{SF}, z_{SF})$. Toutefois, puisque les données mesurées sont des vecteurs, l'intérêt est particulièrement porté sur la rotation entre les repères afin d'obtenir les données de mouvement dans le repère global fixe NF.

L'orientation du repère fixe NF dans le repère local BF par rapport au repère fixe NF peut être exprimée par le quaternion unité $q_{bn}$. Le quaternion unité $q_{bn}$ est décrit avec l'angle de rotation $\psi$ autour de l'axe de rotation défini par le vecteur unitaire de direction $\mathbf{q}_{bn}$ à trois dimensions ou avec un vecteur unitaire à quatre dimensions comme suit :

$$q_{bn} = \begin{bmatrix} \cos(\psi/2) \\ \sin(\psi/2)\mathbf{q}_{bn} \end{bmatrix} = [q_{bn,0} \quad q_{bn,1} \quad q_{bn,2} \quad q_{bn,3}]^T \qquad (1)$$

Dans la présente demande, tous les quaternions sont des quaternions unitaires.

On peut noter que $q_{nb}$ est le conjugué de $q_{bn}$, et que :

$$q_{nb} = \begin{bmatrix} \cos(\psi/2) \\ -\sin(\psi/2)\mathbf{q}_{bn} \end{bmatrix} = [q_{bn,0} \quad -q_{bn,1} \quad -q_{bn,2} \quad -q_{bn,3}]^T \tag{2}$$

Pour ce faire, est définie une matrice de rotation $\mathbf{R}_{nb}$ qui fait passer un vecteur x, exprimé dans le repère BF lié à l'ensemble capteur, au même vecteur exprimé dans le repère global fixe NF par la relation suivante :

$$\mathbf{x}^n = \mathbf{R}_{nb}\,\mathbf{x}^b \tag{3}$$

La matrice de transformation inverse est $\mathbf{R}_{bn} = \mathbf{R}_{nb}^{-1} = \mathbf{R}_{nb}^T$. De manière équivalente, la matrice $\mathbf{R}_{nb}$ est utilisée pour faire passer un vecteur x, exprimé dans le repère global fixe NF, au même vecteur exprimé dans le repère BF lié à l'ensemble capteur.

La matrice de rotation $\mathbf{R}_{nb}$ est liée au quaternion $\mathbf{q}_{bn}$ par la relation suivante :

$$\mathbf{R}_{bn} = \begin{bmatrix} 2q_{bn,0}^2 - 1 + 2q_{bn,1}^2 & 2q_{bn,1}q_{bn,2} - 2q_{bn,0}q_{bn,3} & 2q_{bn,1}q_{bn,3} + 2q_{bn,0}q_{bn,2} \\ 2q_{bn,1}q_{bn,2} + 2q_{bn,0}q_{bn,3} & 2q_{bn,0}^2 - 1 + 2q_{bn,2}^2 & 2q_{bn,2}q_{bn,3} - 2q_{bn,0}q_{bn,1} \\ 2q_{bn,1}q_{bn,3} - 2q_{bn,0}q_{bn,2} & 2q_{bn,2}q_{bn,3} + 2q_{bn,0}q_{bn,1} & 2q_{bn,0}^2 - 1 + 2q_{bn,3}^2 \end{bmatrix} \tag{4}$$

On donne maintenant le modèle des mesures fournies par les capteurs triaxiaux utilisés.

Des accéléromètres mesurent la somme de l'ensemble des accélérations appliquées sur le capteur. Sur Terre, les accéléromètres mesurent, en plus de la gravité terrestre, l'accélération externe subie par le capteur. On définit l'accélération propre comme l'accélération externe subie par le capteur, qui est égale à la dérivée de la vitesse du capteur.

Les accéléromètres ont été utilisés dans de nombreuses applications commerciales, militaires et scientifiques, y compris la navigation inertielle, les systèmes de sécurité des véhicules tels que les airbags, le contrôle de confort de roulement, les plates-formes de stabilisation, et de surveillance des vibrations. Des versions commerciales d'accéléromètres triaxiaux à axes orthogonaux sont largement utilisées pour toutes ces applications, pour lesquelles ils sont montés fixement sur un corps en mouvement.

Un accéléromètre de type microsystème électromécanique ou MEMS comprend, entre autres composants, une masse étalon qui est suspendue élastiquement par un ou plusieurs ressorts de suspension. La masse étalon $m$ se déplace lorsque l'accéléromètre subit une accélération, et son déplacement $x_m$ est proportionnel à la force $f_m$ appliquée à la masse

$$f_m = ma_x = -k\,x_m \tag{5}$$

dans lequel k est la constante du ressort. Le mouvement de la masse étalon m est ensuite converti en un signal électrique ayant un paramètre d'amplitude qui est proportionnel à l'accélération.

L'accélération mesurée inclut la gravité terrestre comme un décalage de l'accélération du corps mesurée, aussi l'accélération du corps mesurée $\mathbf{a}^b$ est liée à l'accélération propre $\mathbf{a}_p^n$ par la relation suivante :

$$\mathbf{a}^b(t) = \mathbf{R}_{bn}(t)\mathbf{a}^n(t) = \mathbf{R}_{bn}(t)(\mathbf{a}_p^n(t) - \mathbf{g}^n) \tag{6}$$

dans laquelle $t$ représente le temps, et $\mathbf{g}^n = [0\ 0\ -g]^T$ représente le vecteur de gravité terrestre avec $g = 9.81$ m/s$^2$.

La Terre peut être considérée, en première approximation, comme un dipôle magnétique, avec le pôle de champ magnétique sud S à proximité du Pôle Nord magnétique de la Terre, et le champ magnétique nord N à proximité du Pôle Sud magnétique de la Terre. Le champ magnétique varie d'un endroit à l'autre et des différences dans le champ magnétique terrestre peuvent être dues à la nature différente des roches. Trois magnétomètres intégrés selon trois axes orthogonaux sont utilisés pour mesurer l'amplitude et l'orientation du champ magnétique, ou en d'autres termes un magnétomètre triaxial.

Bien que les capteurs magnétiques soient un peu plus difficiles à utiliser, ils fournissent des données précises et fiables. Dans de nombreuses applications commerciales, militaires et scientifiques, des magnétomètres sont inclus notamment pour la navigation inertielle. Les magnétomètres détectent également des changements ou des perturbations dans des champs magnétiques qui ont été créés ou modifiés. A partir de ces mesures, il est possible de déduire, par exemple, des informations sur la direction, la présence, la rotation, l'angle, ou la présence de courants électriques. En l'absence de perturbations magnétiques, on suppose que le champ magnétique terrestre $\mathbf{b}^n$ en trois dimensions, exprimé

$$\mathbf{b}^n \approx [0\ \frac{1}{2}\ \frac{-\sqrt{3}}{2}]^T h,$$

dans le repère NF invariant dans le temps. Par exemple, en France, $h \approx 47\,\mu Tesla$. On suppose dans la suite que la composante horizontale de du champ magnétique terrestre $\mathbf{b}^n$ est selon l'axe $y_{NF}$.

Le champ magnétique $\mathbf{b}^b$ mesuré dans le repère local BF est donné par la relation suivante :

$$\mathbf{b}^b(t) = \mathbf{R}_{bn}(t)\left(\mathbf{b}^n + \mathrm{T}^n(t)\right) \tag{7}$$

dans laquelle $\mathrm{T}^n$ désigne la perturbation magnétique.

En l'absence de perturbation magnétique, $\mathbf{b}^n$ est censé être invariant dans le temps: $\mathbf{b}^b$ dépend uniquement de l'orientation du capteur. Toutefois, le champ magnétique terrestre peut être perturbée par des constructions métalliques, des appareils électroniques ou électriques, ... etc. En conséquence, les mesures du magnétomètre sont moins fiables pour déterminer la direction du champ magnétique terrestre dans certaines situations, comme dans un environnement intérieur, et en particulier près de murs ou de planchers ou plafonds, qui comprennent généralement des structures métalliques.

Il est maintenant décrit certaines propriétés de la marche qui est utile dans la suite. Tout d'abord, on définit les différentes phases du cycle de marche telles qu'elles sont définis dans la littérature. L'utilisation d'un système optique de capture de mouvement (de type Codamotion), permet de démontrer quelques propriétés particulières du mouvement de marche. Plus précisément, le mouvement d'une cheville est représenté par un mouvement sensiblement plan. Cette propriété est utilisée dans la présente invention. On analyse également des modèles typiques, observés sur les mesures du capteur au cours d'un cycle de marche.

Dans cette partie, le déplacement spatio-temporel d'une cheville est observé au moyen d'un capteur de mouvement comprenant un accéléromètre, et un magnétomètre ou un gyromètre, monté fixement à proximité de la cheville. On observe les mesures fournies par l'accéléromètre et la variation de l'attitude ou orientation du capteur lors d'un pas.

Lors d'une marche, le cycle de marche est divisé en deux phases :

- une phase d'appui, appelée ST pour "STance phase" en langue anglaise, durant laquelle le pied est en contact avec le sol; et
- une phase de vol, appelée SW pour "SWing phase" en langue anglaise, durant laquelle le pied n'est pas en contact avec le sol

En particulier, le cycle de marche est caractérisé par plusieurs instants caractéristiques notables :

- maintien du pied à plat ou FF pour "Foot Flat" en langue anglaise correspondant à l'instant de la phase d'appui auquel le pied est au sol, et la pointe et le talon touchent le sol ;
- instant où le pied va quitter le sol ou TO pour "Toe Off" en langue anglaise, qui marque la transition entre la phase d'appui ST et la phase de vol SW ;
- instant auquel le pied repart vers l'avant ou MS pour "Mid Swing" durant la phase de suspension, correspondant au milieu de la phase de vol SW ; et
- pose du pied au sol ou HS pour "Heel Strike" en langue anglaise correspondant à l'instant où le talon est en contact avec le sol, représentant la transition entre la phase de vol SW et la phase d'appui ST.

En outre, la vitesse angulaire passe par la valeur zéro à deux reprises au cours d'une phase de vol SW, respectivement avant et après l'instant MS. Un instant de passage par zéro de la vitesse angulaire, et un instant de pose de pied au sol HS sont utilisés par la suite.

Pour étudier le comportement de la cheville lors d'une promenade, à l'aide d'un système de capture de mouvement optique en trois dimensions, nommé Coda CX1 par la société Charnwood Dynamics. Son unité principale de mesure contient trois caméras alignées reliées fixement, qui suivent la position d'un certain nombre de marqueurs actifs (diodes électroluminescentes ou LED infrarouge) en temps réel. La sortie du système est enregistrée dans un fichier texte contenant les coordonnées en trois dimensions des marqueurs actifs $\mathbf{p}_{LED}^{(l)}$ où $l$ désigne l'indice de temps. La précision spatiale est de l'ordre de 1 mm. Avec trois marqueurs sur un corps rigide, il est également possible de suivre l'orientation du corps. Ainsi, trois marqueurs sont fixés sur la cheville d'un piéton qui marche devant la caméra.

Les échantillons de position obtenus sont donnés dans le repère du Coda CX1, qui est lié au repère NF et est défini par la position des LED au début de la marche. Cependant, puisque nous ignorons cette relation et pour des raisons de clarté, on fait tourner les échantillons de position afin de corriger ou redresser la trajectoire de la cheville de manière à avoir une foulée horizontale et un mouvement dans un plan vertical.

Par conséquent, on détermine v, le vecteur unitaire orthogonal au plan du mouvement, avec la plus petite translation par une décomposition en valeurs singulières ou SVD pour "Singular Value Decomposition" en langue anglaise, appliquée à l'ensemble des échantillons de position à trois dimensions.
$$\mathbf{p}_{LED} = \left[ \mathbf{p}_{LED}^{(l_1)} \; \cdots \; \mathbf{p}_{LED}^{(l_2)} \right]^T .$$

$l_1$ et $l_2$ sont des indices temporels, correspondant respectivement au début et à la fin d'un pas. Le vecteur v est le vecteur singulier droit, i.e. le vecteur propre associé à la plus petite valeur singulière ou valeur propre. Le vecteur v est colinéaire à l'axe $x_{SF}$, et on suppose que la direction de la foulée est colinéaire à l'axe $y_{SF}$.

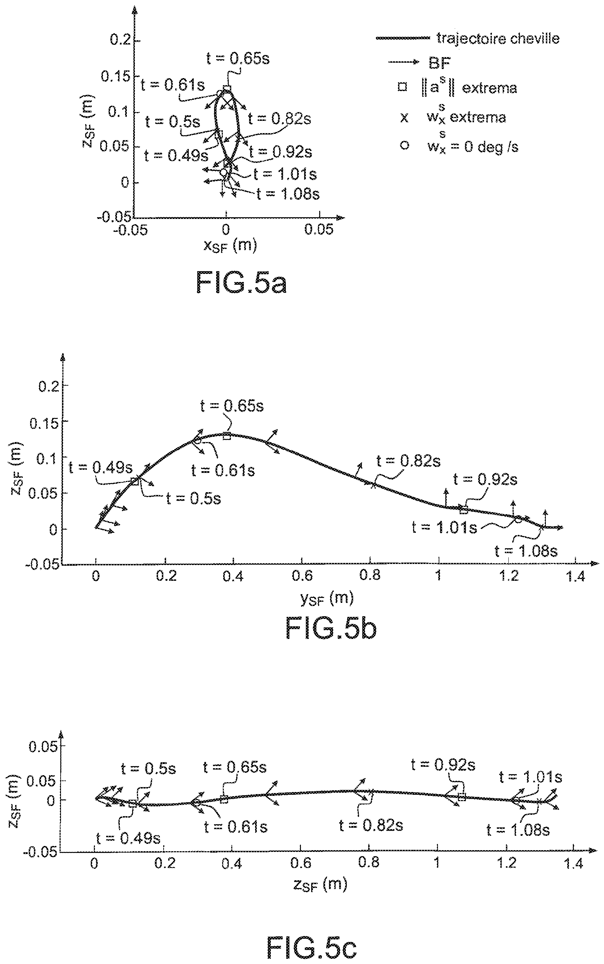

Les figures 5a, 5b et 5c montrent la trajectoire de la cheville droite d'un utilisateur durant un pas. La vitesse moyenne d'un piéton est approximativement de 0,6 m/s. Comme on le voit respectivement sur les figures 5a, 5b et 5c, la variation selon l'axe $x_{SF}$ est inférieure à 1 cm, tandis que les variations selon les axes $y_{SF}$ et $z_{SF}$ sont respectivement supérieures à 130 cm et 13 cm. La trajectoire représente le mouvement d'une LED capturé par une caméra. La LED est fixée à la cheville d'un piéton projetée sur les plans $(x_{SF}, z_{SF})$, $(y_{SF}, z_{SF})$, et $(y_{SF}, x_{SF})$.

Les instants où le pied va quitter le sol TO et de pose du pied au sol HS peut être déterminés avec précision à partir des deux minimum de vitesse angulaire mesurés.

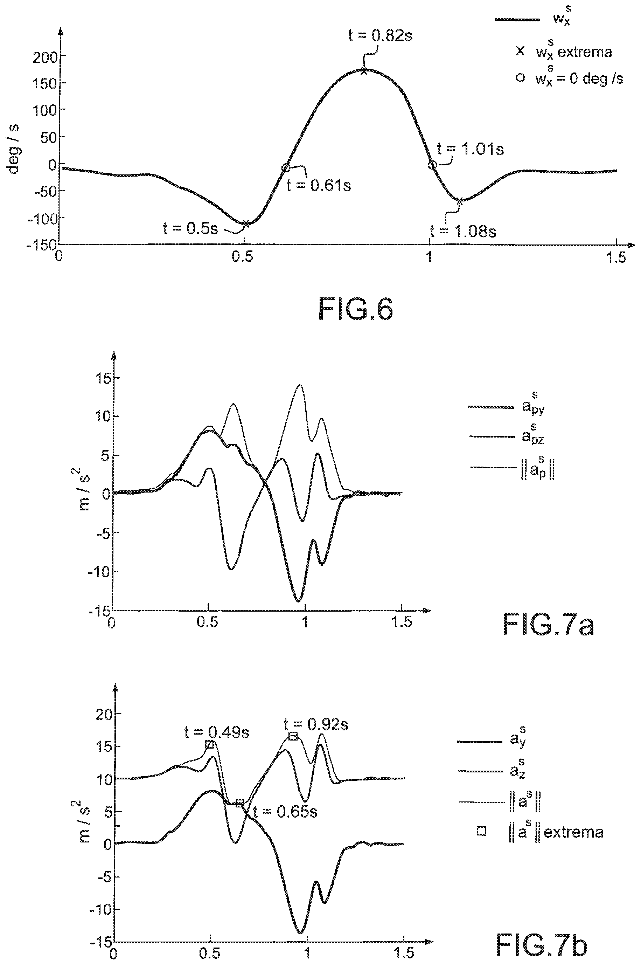

La figure 6 montre les valeurs extrema de la vitesse angulaire selon l'axe $x_{SF}$ déduites des positions de la LED fournies par le système Coda CX1 : le passage par zéro, les instants TO et HS. Comme la variation de la trajectoire de la cheville selon l'axe $x_{SF}$ est négligeable, on procède de l'analyse dans le plan du pas $(y_{SF}, z_{SF})$. La figure 7a montre l'accélération propre $a_{py}^s$ et $a_{pz}^s$ pendant l'intervalle de temps du pas. Les données sont obtenues à partir de la trajectoire du pied des figures 5a, 5b et 5c et lissées avec un filtre passe-bas de fréquence de coupure environ égale à 20 Hz. La figure 7b montre l'accélération pour la même trajectoire que celle des figures5a, 5b et 5c, dans le repère SF par dérivation des mesures fournies par le système Coda CX1. La différence entre les figures 7a et 7b, est que dans le second cas, $a_z^s = a_{pz}^s + g$ et les données sont lissées avec un filtre passe-bas de fréquence de coupure environ égale à 10Hz au lieu de 20Hz. Il est possible de voir l'impact de la bande de la fréquence de coupure (à expliquer). La figure 7b montre trois instants critiques qui caractérisent le modèle $\|\mathbf{a}^s\|$ (à expliquer) quand les courbes passent par un premier maximum à t=0.49 s nommé $Max_1$, un minimum à l'instant t=0.65 s nommé $Min$ et un second maximum à t=0.92 s, nommé $Max_2$. En détectant ces valeurs extrema, il est facile de détecter les pas des piétons, comme explicité dans la suite de la description. Les instants correspondant à ces valeurs extrema sont également visibles sur les figures 5a, 5b et 5c, ainsi que les valeurs critiques de $\omega_x^s$.

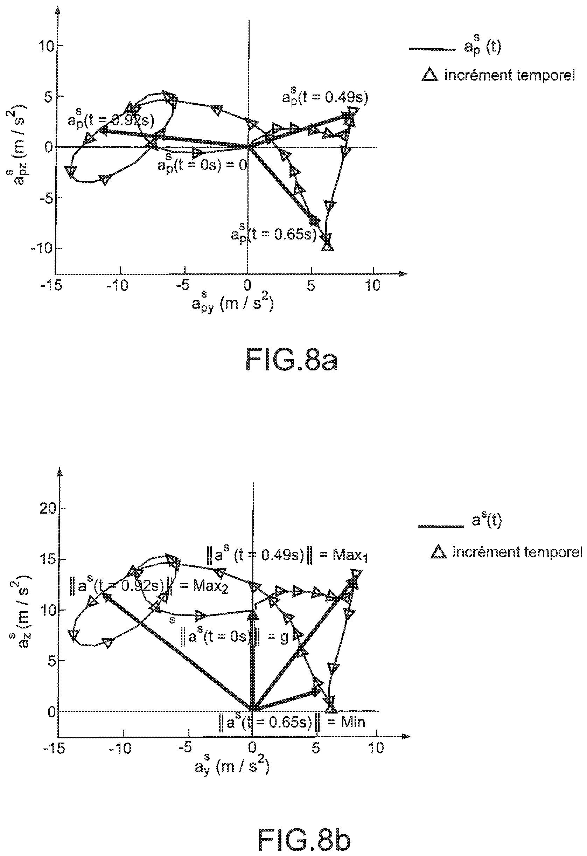

Pour illustrer les variations des mesures de l'accéléromètre, les figures 8a et 8b montrent l'accélération dans le plan $(y_{SF}, z_{SF})$ et les instants associés. La figure 8a représente l'accélération en deux dimensions de la figure 7a. La figure 8b représente l'accélération de la Figure 7b dans le repère SF.

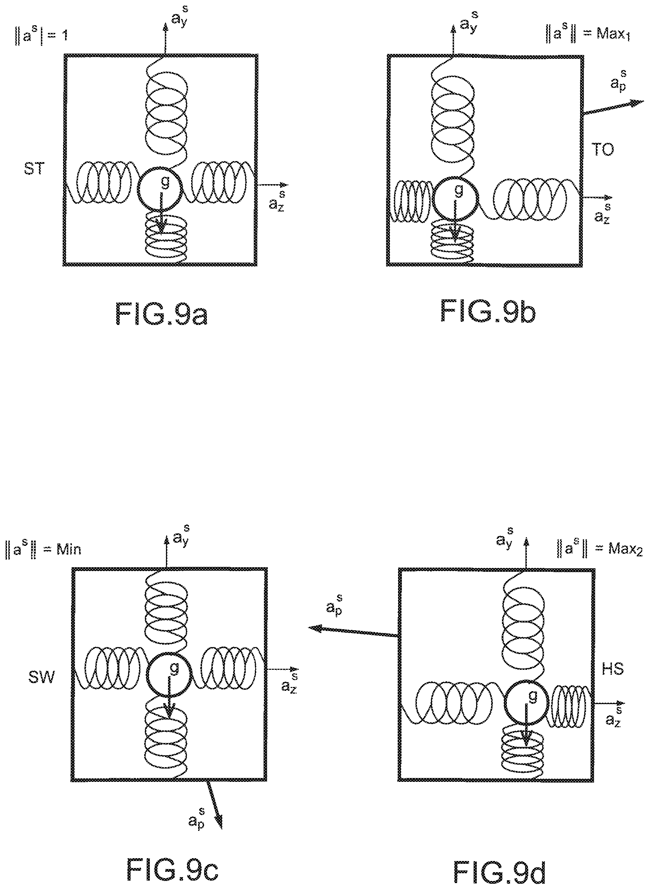

Comme indiqué précédemment, un accéléromètre à deux axes de mesure peut être considéré comme une masse étalon fixée à des ressorts. Les figures 9a, 9b, 9c et 9d illustrent le comportement d'un accéléromètre monté sur une cheville d'un piéton dans différentes situations. L'accéléromètre est représenté à quatre moments qui font référence aux phases ST et SW et aux instants TO et HS. Dans la phase d'appui ST, figure 9a, l'accélération propre est nulle et l'accéléromètre ne mesure que l'accélération de la pesanteur. Les trois valeurs extrema de $\mathbf{a}^s$ représentées sur la figure 8b correspondent aux instants TO, MS, et HS. Cela peut s'expliquer par l'orientation et la norme de l'accélération propre à ces instants comme sur la figure 8a, qui sont illustrés sur les figures 9a, 9b, 9c et 9d.

De nombreuses méthodes de détection de pas sont connues. Elles sont, par exemple, basées sur des transformations de Fourier, le comptage de passages par zéro de la sortie d'un accéléromètre ou le comptage des points qui dépassent un seuil ("In step with INS: navigation for the blind, tracking emergency crews" de Q. Ladetto and B. Merminod, GPS World, Vol. 13(10), pp. 30-38, 2002).

Même si ces méthodes sont simples, elles peuvent souffrir d'erreurs importantes lorsque les utilisateurs marchent dans des escaliers, le long d'un terrain en pente, ou quand lorsqu'ils courent ou traînent les pieds. D'autres méthodes précises sont basées sur la variation angulaire du pied et ils peuvent estimer chaque phase d'un pas ("A Magnetometer-Based approach for studying human movements" de S. Bonnet and R. Heliot, IEEE Transactions on Biomédical Engineering, Vol. 54(7), pp. 1353-1355, 2007).

Dans la présente invention, on met en oeuvre une détection simple de pas. Une solution pratique pour détecter le cycle de la marche est d'attacher le capteur sur la cheville du piéton. Puis, grâce à des mesures d'accélération ou de vitesse angulaire, non détecte les pas de manière robuste et fiable.

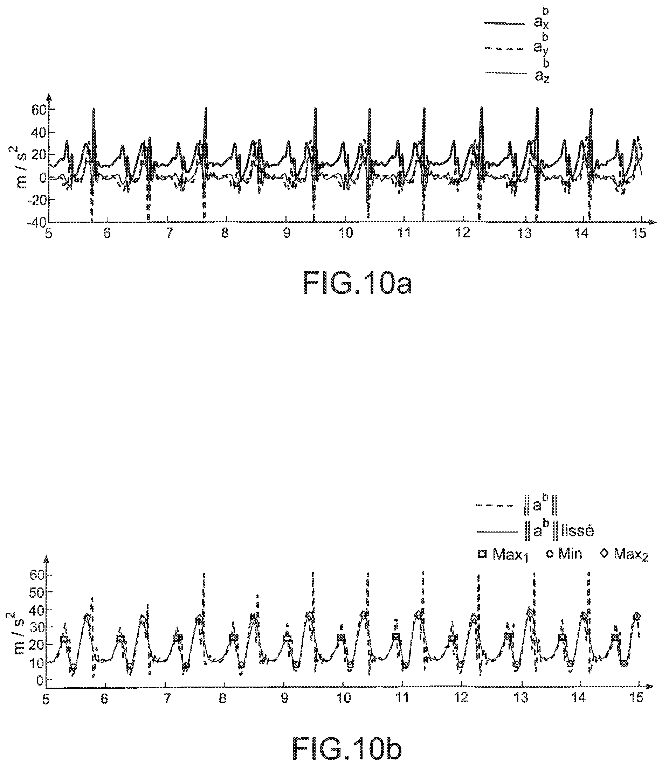

Lors de son déplacement, le pied d'un piéton suit quasiment la même forme de trajectoire pour les différentes pas. En fait, cela est vrai même pour des piétons différents, qui ne présentent pas de trouble de la marche. La norme $\|\mathbf{a}^n\|$ de l'accélération mesurée est considérée et son modèle est représenté sur la figure 7b. Les données brutes mesurées contiennent, en plus du bruit, des valeurs importantes à hautes fréquences en particulier lors de la pose du pied au sol HS. Ces composantes hautes fréquences diffèrent d'un pas à l'autre, également pour un même piéton, ainsi que pour une même vitesse de marche. Aussi, il est commode de lisser les données avant de procéder à la détection de pas. Les figures 10a et 10b montrent l'amplitude de l'accélération fournie par un accéléromètre triaxial fixé à la cheville d'un piéton, pour quelques pas de marche. Un exemple de données d'accélération lissées est illustré sur les figures 7a, 7b, 8a, et 8b. Même si la trajectoire la cheville est similaire pour les différents pas, les sorties du capteur dépendent de l'orientation du capteur durant le pas. Ce problème est principalement du à l'orientation du capteur à la cheville au début (du pas?). Pour surmonter le problème de la variation temporelle de l'orientation du capteur à la cheville, il est possible d'utiliser simplement l'amplitude des mesures de l'accéléromètre dans le repère BF ("Robust step détection method for pedestrian navigation systems", de H.-J. Jang, J.W. Kim, et D.H. Hwang, Electronics Letters, 43(14), 2007, selon la relation suivante :

$$\left\| \mathbf{a}^b(t) \right\| = \left\| \mathbf{R}_{bn}(t)\mathbf{a}^n(t) \right\| = \left\| \mathbf{a}^n(t) \right\| \qquad (7)$$

Ainsi, il est possible d'utiliser $\|\mathbf{a}^b(t)\|$ parce que le modèle est égal au modèle de $\|\mathbf{a}^n(t)\|$ (cf. le modèle des figures 7a et 7b) et ne dépend pas de l'orientation du capteur. En revanche, pour la vitesse angulaire, on peut utiliser $\omega_x^n$ (cf. le modèle de la figure 6).

Une solution est de comparer ce modèle à un modèle. Malheureusement, le modèle est souvent une version à échelle réduite en temps et en amplitude (à clarifier), qui nécessite de nombreux traitements du signal. Une solution pratique consiste à détecter les valeurs extrema du modèle par exemple à partir d'un passage de seuil.

Le but n'est pas seulement la détection de pas, mais également la détermination du début et de la fin du pas. Grâce a cette détermination, on peut limiter les dérives dues à l'intégration en fixant la vitesse nulle au début et à la fin du pas.

On met en oeuvre un système de suivi de pose de jambe utilisant seulement deux capteurs fixés respectivement à la cheville droite et à la cheville gauche d'un piéton. Les capteurs peuvent être des capteurs 3A3M (accéléromètre triaxial et magnétomètre triaxial), 3A3G (accéléromètre triaxial et gyromètre triaxial) ou 3A3G3M (accéléromètre triaxial et magnétomètre triaxial et gyromètre triaxial). L'idée est d'utiliser l'hypothèse de marche sagittale (dans un plan vertical orienté dans le sens antéropostérieur sur la ligne médiane du corps), afin de réduire le nombre de degrés de liberté pour le modèle. En outre, on utilise un modèle cinématique des jambes, semblable à ceux utilisés en robotique. L'hypothèse de plan sagittal et le modèle biomécanique des jambes permettent de résoudre le problème d'estimation du mouvement des membres inférieurs et de navigation avec un nombre de capteurs réduit.

Dans la suite de la description, on décrit la modélisation cinématique des jambes du piéton durant une marche. Les paramètres qui définissent le modèle sont prévus pour exploiter les relations entre chaque partie de la chaîne articulée CH du système et pour les phases de marche. Ensuite, un exemple de mouvement des jambes lors d'une marche est décrit en utilisant les paramètres de simulation. On décrit également comment déterminer les paramètres du modèle, étant donné les données inertielles et la trajectoire du piéton permettant le suivi spatio-temporel du mouvement des jambes du piéton.

On modélise la partie des jambes du piéton située entre la taille (les hanches) et la cheville, comme dans l'exemple décrit précédemment, dans la description de la figure 1

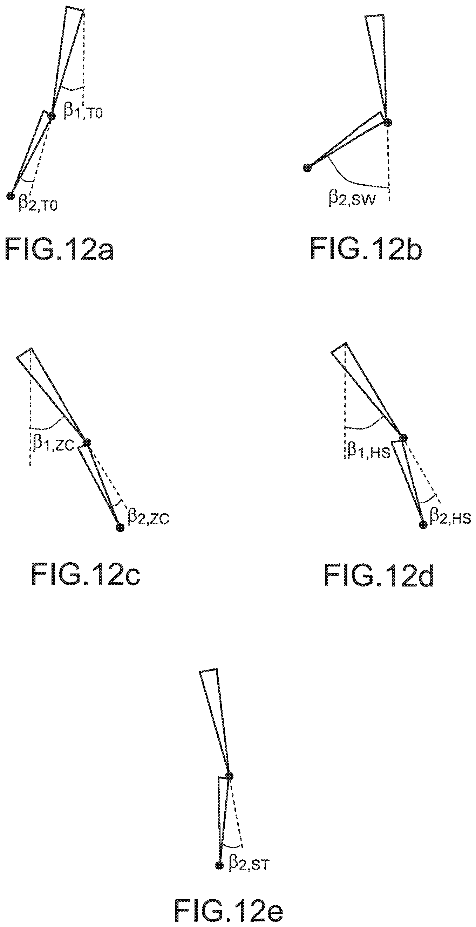

Une manière de modéliser les variations des angles des jambes est de les interpoler entre les valeurs extrémales. Les figures 12a, 12b, 12c, 12d et 12e représentent des valeurs critiques de $\beta_1$ et $\beta_2$ durant une marche. $\beta_1$ croît de sa valeur minimum à sa valeur maximum entre les instants TO où le pied va quitter le sol et la pose du pied au sol HS. La valeur décroît ensuite pour atteindre à nouveau sa valeur minimum à l'instant suivant TO où le pied va quitter le sol. $\beta_2$ est caractérisé par quatre valeurs extrémales au cours d'un cycle de marche. Au cours de la phase de vol SW, $\beta_2$ varie de $\beta_{2,TO}$ à $\beta_{2,HS}$, et atteint son plus faible minimum $\beta_{2,SW}$ (i.e. à la flexion maximum du genou), et $\beta$ atteint son maximum $\beta_{ZC}$. Au cours de la phase d'appui ST, $\beta_2$ varie de $\beta_{2,HS}$ à $\beta_{2,TO}$, durant laquelle il atteint un autre extremum local $\beta_{2,ST}$.

On montre maintenant comment le modèle proposé ci-dessus peut imiter la marche de l'homme. On simule les angles en fixant les paramètres du modèle de manière à respecter les angles critiques précédemment représentés aux figures 12a, 12b, 12c, 12d et 12e. Les angles $\{\beta_{1,TO}, \beta_{2,TO}\}$ et $\{\beta_{1,HS}, \beta_{2,HS}\}$ correspondent respectivement aux angles correspondant aux instants de TO où le pied va quitter le sol et de HS de la pose du pied au sol, tandis que les angles $\{\beta_{1,SW}, \beta_{2,SW}\}$ et $\{\beta_{1,ST}, \beta_{2,ST}\}$ correspondent respectivement aux extrema locaux des angles au cours des phases de vol SW et d'appui ST, mais pas nécessairement à ceux des instant auxquels le pied repart vers l'avant MS et de maintien du pied à plat FF. L'occurrence de ces angles est examinée dans la suite de la description.

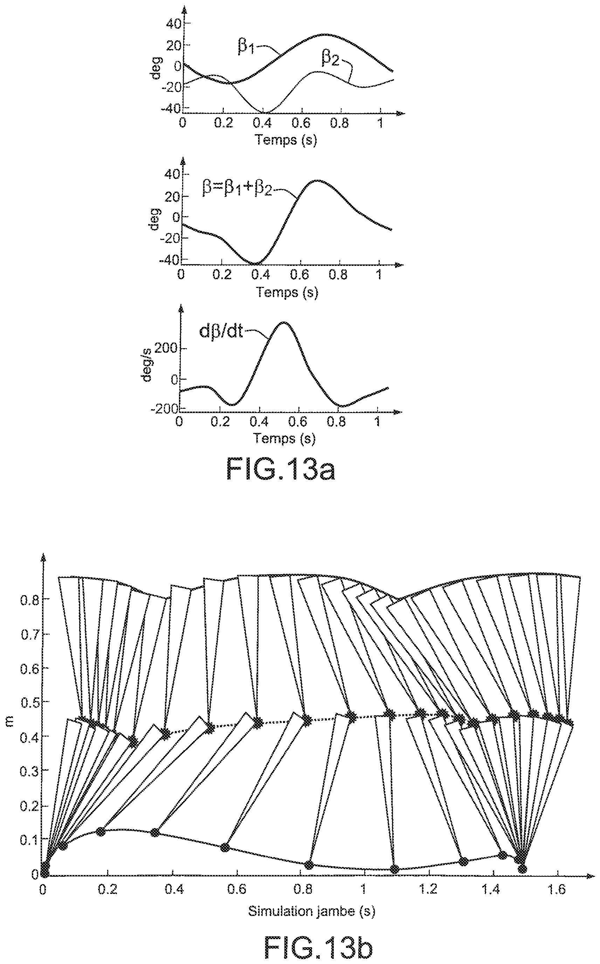

Une solution classique pour modéliser l'évolution temporelle de ces angles est d'utiliser des fonctions cosinus. A titre d'exemple, les figures 13a et 13b représentent des angles simulés $\beta_1$ et $\beta_2$. L'angle $\beta_1$ est simulé avec un cosinus démarrant à l'instant de pose du pied au sol HS.

$$\text{HS} \rightarrow \text{ST} \rightarrow \text{TO} \rightarrow \text{SW} \rightarrow \text{HS} \qquad \beta_1(t) = \beta_{1,HS} + \frac{\beta_{1,TO} - \beta_{1,HS}}{2}\left(1 - \cos(2\pi\frac{t}{T_{gait}})\right) \quad (9)$$

$T_{gait} = T_{Stance} + T_{Swing}$ est la durée d'un cycle de marche. L'angle $\beta_2$ est simulé en utilisant quatre cosinus consécutifs, chacun représentant la partie du modèle des angles entre deux extrema consécutifs :

$$
\begin{aligned}
\text{HS} \rightarrow \text{ST}: \quad \beta_2(t) &= \beta_{2,HS} + \frac{\beta_{2,ST} - \beta_{2,HS}}{2}\left(1 - \cos(\pi\frac{t}{T_{HS,ST}} + \beta_{12})\right) \\[2mm]
\text{ST} \rightarrow \text{TO}: \quad \beta_2(t) &= \beta_{2,ST} + \frac{\beta_{2,TO} - \beta_{2,ST}}{2}\left(1 - \cos(\pi\frac{t - T_{HS,ST}}{T_{ST,TO}} + \beta_{12})\right) \\[2mm]
\text{TO} \rightarrow \text{SW}: \quad \beta_2(t) &= \beta_{2,TO} + \frac{\beta_{2,SW} - \beta_{2,TO}}{2}\left(1 - \cos(\pi\frac{t - T_{HS,TO}}{T_{TO,SW}} + \beta_{12})\right) \\[2mm]
\text{SW} \rightarrow \text{HS}: \quad \beta_2(t) &= \beta_{2,SW} + \frac{\beta_{2,HS} - \beta_{2,SW}}{2}\left(1 - \cos(\pi\frac{t - T_{HS,SW}}{T_{SW,HS}} + \beta_{12})\right)
\end{aligned}
\qquad (10)
$$

$T_{HS,ST}$, $T_{ST,TO}$, $T_{TO,SW}$ et $T_{SW,HS}$ désignent les intervalles de temps séparant les extrema correspondants aux indices. L'angle $\beta_{12}$ est un angle de phase entre $\beta_1$ et $\beta_2$. L'angle $\beta_{12}$ est explicité par la suite dans la description, où sa dépendance avec la pente de la marche est démontrée.

Les figures 13a et 13b illustrent une simulation de la marche dans laquelle les angles des jambes gauche et droite suivent les équations (9) et (10) avec un temps de retard relatif $T_{gait}/2$ entre les angles gauche et droit. Les figures 13a et 13b représentent respectivement les variations temporelles angulaires et le mouvement de jambes du modèle biomécanique. En l'espèce, on a pris $T_{HS,ST} = T_{ST,TO} = T_{TO,SW} = T_{SW,HS} = T_{gait}/4$.

Les extrema sont donnés pour cette marche simulée dans le tableau suivant, ainsi que $\beta_{12}$. $L_F$ et $L_T$ représentent la longueur des segments représentant respectivement le fémur et le tibia.

| $\beta_{1,TO}$ | $\beta_{1,HS}$ | $\beta_{2,TO}$ | $\beta_{2,SW}$ | $\beta_{2,HS}$ | $\beta_{2,ST}$ | $\beta_{12}$ | $L_F$ | $L_T$ |
|---|---|---|---|---|---|---|---|---|
| -15° | 30° | -10° | - 45° | -5° | -25° | 20° | 43 c m | 45 c m |

Enfin, en supposant que la cheville ne bouge pas au cours d'une phase d'appui ST (en supposant la vitesse de la cheville nulle), les coordonnées ($y_W^s$, $z_W^s$) peuvent être calculées. En effet, connaissant la position d'un point de la chaîne (ici la cheville au sol) et tous les angles articulaires (donnés par les équations 9 et 10 et la position dans le cycle de marche), il est possible de déterminer la position de n'importe quel point de la chaîne (en l'espèce la hanche).

Sur les figures 13a et 13b, est représentée la trajectoire de la cheville calculée à partir des angles simulés. Il est clair que cette trajectoire simulée est typique d'une cheville de piéton. On peut remarquer que ces angles sont modélisés en utilisant les angles critiques des figures 12a, 12b, 12c, 12d, et 12e. On suppose dans la suite que les angles critiques des figures 12a, 12b, 12c, 12d, et 12e sont représentatifs d'une marche typique.

Il y a deux manières de déterminer la pente lors d'une marche à partir des angles $\beta_1$ et $\beta_2$.

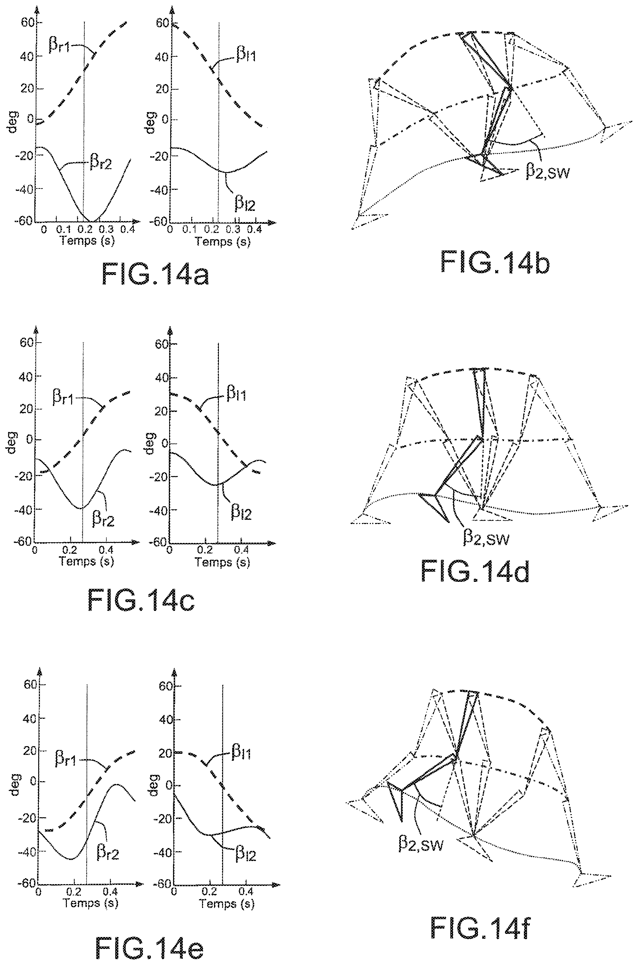

La première manière de déterminer la pente (ou angle entre l'horizontale et le vecteur du déplacement au cours d'un pas) repose uniquement sur les extrema de $\beta_1$ qui augmentent avec la pente ou l'inclinaison. Les figures 14a, 14c et 14e représentent trois valeurs de $\beta_1$ associées à trois pentes représentées respectivement sur les figures 14b, 14d, et 14f. Pour déterminer la pente lors de la marche, l'angle absolu $\beta_1$ par rapport à la verticale est nécessaire.

La deuxième manière de déterminer la pente est d'exploiter la relation entre l'angle de phase $\beta_{12}$ dans les équations (10) et la pente du pas. Dans la pratique, la phase $\beta_{12}$ est quasiment équivalente à l'intervalle de temps entre l'instant auquel le pied repart vers l'avant MS et la valeur minimale de $\beta_2$.

Les figures 14a, 14b, 14c, 14d, 14e, et 14f représentent les modèles d'évolution angulaires pour différentes pentes. Lorsque la pente change, le moment où la valeur minimale de $\beta_2$ est atteinte pendant la phase de vol SW ($\beta_{2,SW}$) change par rapport à l'instant auquel le pied repart vers l'avant MS. Aussi, l'occurrence de $\beta_{2,SW}$ dépend de l'angle de la pente. Par exemple, les figures 14a, 14b, 14c, 14d, 14e, et 14f montrent que $\beta_{2,SW}$ se produit :

- à la fin de la phase de vol SW (par exemple après l'instant MS auquel le pied repart vers l'avant, comme sur la figure 14a) avec une montée (cf figure 14b) ;
- au milieu (par exemple à proximité de l'instant MS auquel le pied repart vers l'avant, comme sur la figure 14c) avec une pente nulle (cf figure 14d) ;
- au début de la phase de vol SW (par exemple avant l'instant MS auquel le pied repart vers l'avant, comme sur la figure 14e) avec une descente (cf figure 14f).

En fait, lorsqu'un piéton marche suivant une pente donnée, sa taille (ses hanches) suit une trajectoire avec une pente constante. Comme le piéton doit déplacer son pied au dessus du sol, la valeur minimale $\beta_{2,SW}$ est atteinte lorsque la ligne reliant sa taille et son pied en l'air devient orthogonale au sol.

Comme indiqué précédemment, le modèle biomécanique à deux dimensions de la figure 1 a six degrés de liberté. On montre comment estimer/prévoir les six paramètres (i.e. $\beta_{l1}$, $\beta_{l2}$, $\beta_{r1}$, $\beta_{r2}$, $y_W^s$, $z_W^s$) en utilisant les angles $\beta_r$ et $\beta_l$ des tibias droit et gauche définis dans la relation 1 a. Enfin, les résultats expérimentaux utilisant deux magnétomètres fixés aux chevilles montrant l'orientation et la position des jambes du piéton lors d'une marche sont présentés.

Généralement, lors d'une marche, au moins un des pieds touche le sol. On peut donc supposer que la vitesse de la cheville est égale à zéro pendant la phase d'appui ST. Avec cette hypothèse et connaissant le mouvement de rotation des jambes, il est possible de déterminer la vitesse de la taille (des hanches). Par conséquent, si l'ensemble de quatre angles $\{\beta_{r1},\beta_{r2},\beta_{l1},\beta_{l2}\}$ est connu, il est alors possible de calculer ($y_W^s$, $z_W^s$) en fonction des angles de la jambe qui est en phase d'appui ST. Soit, ($x_{RK}^s$, $y_{RK}^s$, $z_{RK}^s$), ($x_{LK}^s$, $y_{LK}^s$, $z_{LK}^s$), ($x_{RA}^s$, $y_{RA}^s$, $z_{RA}^s$) et, ($x_{LA}^s$, $y_{LA}^s$, $z_{LA}^s$), respectivement les coordonnées des genoux droit et gauche, des chevilles droite et gauche, où toutes les coordonnées $x^s$ sont égales à zéro. La pose des jambes est construite et les coordonnées sont calculées comme suit :

$$
\begin{array}{llll|lll}
y_W^{s(l)} & = & y_W^{s(l-1)} + \delta_y^{(l)} & & z_W^{s(l)} & = & z_W^{s(l-1)} + \delta_z^{(l)} \\
y_{RK}^{s(l)} & = & y_W^{s(l)} + L_F \sin \tilde{\beta}_{r1}^{(l)} & & z_{RK}^{s(l)} & = & z_W^{s(l)} - L_F \cos \tilde{\beta}_{r1}^{(l)} \\
y_{LK}^{s(l)} & = & y_W^{s(l)} + L_F \sin \tilde{\beta}_{l1}^{(l)} & & z_{LK}^{s(l)} & = & z_W^{s(l)} - L_F \cos \tilde{\beta}_{l1}^{(l)} \\
y_{RA}^{s(l)} & = & y_{RK}^{s(l)} + L_T \sin \beta_r^{(l)} & & z_{RA}^{s(l)} & = & z_{RK}^{s(l)} - L_T \cos \beta_r^{(l)} \\
y_{LA}^{s(l)} & = & y_{LK}^{s(l)} + L_T \sin \beta_l^{(l)} & & z_{LA}^{s(l)} & = & z_{LK}^{s(l)} - L_T \cos \beta_l^{(l)}
\end{array}
\qquad (11)
$$

Alors que les valeurs « $\beta$ » sont obtenues avec les moyens de mesure MES_O, les valeurs notées « $\tilde{\beta}$ » sont déduites en utilisant les modèles d'évolution EVOL (équations 9 et 10) et certaines hypothèses qui lient $\beta_1$ et $\beta_2$ à $\beta$ (équation 19 par exemple). ($\delta_y^{(l)}$, $\delta_z^{(l)}$) définit une translation de la taille (des hanches) de manière à vérifier la phase d'appui ST. Aussi :

- si la cheville droite est en phase d'appui ST:

$$
\begin{array}{lll}
\delta_y^{(l)} & = & y_{RA}^{s(l-1)} - y_W^{s(l-1)} - L_F \sin(\tilde{\beta}_{r1}^{(l)}) - L_T \sin(\beta_r^{(l)}) \\
\delta_z^{(l)} & = & z_{RA}^{s(l-1)} - z_W^{s(l-1)} + L_F \cos(\tilde{\beta}_{r1}^{(l)}) + L_T \cos(\beta_r^{(l)})
\end{array}
\qquad (12)
$$

- si la cheville gauche est en phase d'appui ST:

$$\begin{aligned}
\delta_y^{(l)} &= y_{LA}^{s(l-1)} - y_W^{s(l-1)} - L_F \sin(\tilde{\beta}_{l1}^{(l)}) - L_T \sin(\beta_l^{(l)}) \\
\delta_z^{(l)} &= z_{LA}^{s(l-1)} - z_W^{s(l-1)} + L_F \cos(\tilde{\beta}_{l1}^{(l)}) + L_T \cos(\beta_l^{(l)})
\end{aligned} \qquad (13)$$

Comme les coordonnées cartésiennes à deux dimensions de la taille du piéton peuvent être calculées une fois les quatre angles des jambes déterminés, le problème est réduit à quatre degrés de liberté.

La pose des jambes à quatre degrés de liberté peut être déterminée par l'ensemble des quatre angles $\{\beta_{r1}, \beta_{r2}, \beta_{l1}, \beta_{l2}\}$. Le plus simple moyen de déterminer ces angles est d'attacher un capteur de mouvement à chaque segment du modèle biomécanique (deux fémurs et deux tibias). Cependant, puisque nous supposons que, lors d'une marche, les segments tournent selon un axe invariant orthogonal et se déplacent dans un plan orthogonal à cet axe, il n'est pas nécessaire de déterminer l'orientation en trois dimensions pour construire le modèle biomécanique de la figure 11. Ainsi, la solution minimale consiste à positionner un magnétomètre à deux axes de mesures 2M dans le plan orthogonal à l'axe de rotation ou un gyromètre à un axe de mesure 1G parallèle à l'axe de rotation (médio latéral dans le cas de la marche normale). Une autre solution consiste à s'affranchir de l'orientation du capteur par rapport au segment en utilisant un magnétomètre triaxial 3M ou un gyromètre triaxial 3G attaché à chaque partie des jambes du piéton (i.e. les quatre segments illustré sur la figure 11). On suppose également que le capteur de mouvement tourne autour d'un axe invariant dans le temps, il est possible de déterminer l'axe de la rotation. Ainsi, l'angle de chaque segment du modèle biomécanique peut être déterminé.

En outre, la réduction du nombre de capteurs est plus pratique pour une utilisation quotidienne. De plus, cela réduit le coût et la consommation électrique de l'ensemble du système de navigation. Par conséquent, on utilise deux capteurs de mouvement fixés sur les chevilles gauche et droite permettant le suivi de pose des jambes du piéton. Cette méthode a été appliquée dans un environnement extérieur où les magnétomètres fixés à une cheville ne sont pas perturbés. Ainsi, il est possible de déterminer la position à partir du magnétomètre monté sur la cheville. Dans un suivi de pose des jambes du piéton en intérieur, un magnétomètre près du sol est fortement perturbé. Ainsi, un autre capteur de mouvement peut être utilisé pour déterminer la position. Par exemple, un troisième capteur de mouvement, monté sur la taille du piéton peut être utilisé pour suivre la position du piéton.

Dans la suite on se focalise sur l'estimation de la pose des jambes du piéton utilisant les deux capteurs de mouvement montés sur les chevilles.

La détermination de la pose des jambes pour le modèle biomécanique de la figure 11 nécessite la connaissance des quatre angles. Par conséquent, il faut déduire quatre degrés de liberté à partir de $\beta_l$ et $\beta_r$. Une solution consiste à réduire les quatre degrés de liberté du modèle à deux degrés de liberté, le nombre d'angles disponibles. Par exemple, il est montré précédemment la possibilité d'établir des fonctions du temps pour les angles $\{\beta_{r1}, \beta_{r2}, \beta_{l1}, \beta_{l2}\}$ utilisant les fonctions cosinus. Grâce à ces fonctions, un seul angle est nécessaire pour déterminer l'ensemble de la pose des jambes, réduisant le modèle à un degré de liberté au lieu de quatre. Cependant, cette méthode manque de précision. En outre, la pente de la marche change l'occurrence temporelle et la valeur des extrema d'angles $\{\beta_{1,TO}, \beta_{1,HS}, \beta_{2,TO}, \beta_{2,SW}, \beta_{2,HS}, \beta_{2,ST}\}$ tel que décrit précédemment, ce qui rend une relation universelle très complexe à établir.

Une autre solution consiste à exploiter les informations implicites inclues dans les angles $\beta_l$ and $\beta_r$, en plus les angles eux-mêmes. Ces informations permettent de prévoir quatre angles à partir des deux angles inconnus en s'appuyant sur des relations d'angles relatives à la marche d'un piéton. On propose d'interpoler les angles $\beta_{r1}$ et $\beta_{l1}$ en s'appuyant sur les valeurs critiques et leurs instants d'occurrence, et les passages par zéro des vitesses angulaires $d\beta_r/dt$ et $d\beta_l/dt$, où $Max_1$ et $Max_2$ sont respectivement atteints à l'instant TO où le pied va quitter le sol et à l'instant HS de pose du pied au sol, tandis que les passages par zéro correspondent à la valeur maximum de $\beta$.

On détermine maintenant les angles $\beta_r$ et $\beta_l$. On peut utiliser un magnétomètre à au moins deux axes de mesure 2M ou un gyromètre à au moins deux axes de mesure 2G ou un gyromètre à un axe de mesure G pour déterminer les angles $\beta_r$ et $\beta_l$. Quand on utilise un magnétomètre triaxial 3M ou un gyromètre triaxial 3G, il est alors possible de s'affranchir de l'orientation du capteur par rapport au segment et de déterminer $\beta_l$ et $\beta_r$ en estimant au préalable l'axe de la rotation du segment dans le repère du capteur. Notons ($\mathbf{v}_1^b$, $\mathbf{v}_2^b$, $\mathbf{v}_3^b$) l'ensemble des trois vecteurs singuliers issus de la SVD de la matrice des mesures (lignes=ensemble des mesures durant le pas et colonne = les 3 axes), où ($\mathbf{v}_1^b$, $\mathbf{v}_2^b$) sont associés au plan de la marche et $\mathbf{v}_3^b$ l'axe de la rotation sensiblement invariant dans le temps exprimé dans le repère du capteur BF. Avec les mesures $\omega^b$ du gyromètre, on peut obtenir la vitesse angulaire autour de l'axe $\mathbf{v}_3$, selon la relation suivante :

$$\omega_{\mathbf{v}_3}(t) = \omega^b(t) \cdot \mathbf{v}_3^b \qquad (14)$$

Dans laquelle "·" désigne le produit scalaire. Ensuite, la vitesse angulaire $\omega_{v3}(t)$ est intégrée pour donner la relation suivante :

$$\beta(t) = \beta(t_0) + \int_{t_0}^{t} \omega_{\mathbf{v}_3}(t)dt \qquad (15)$$

$t_0$ est l'instant FF de maintien du pied à plat au cours de la phase d'appui ST, déterminé par une méthode de détection permettant l'identification des différentes phases de la marche (EP 2106747) $\beta(t_0)$ est l'angle du tibia par rapport à la verticale à l'instant $t = t_0$.

De même, $\beta(t)$ peut être calculé en utilisant $\mathbf{b}^b(t)$ par la relation suivante :

$$\beta(t) = \beta(t_0) + \beta_{\mathbf{b}}(t) \qquad (16)$$

dans laquelle :

$$\beta_{\mathbf{b}}(t) = \mathrm{sign}(|[\mathbf{v}_3^b \quad \mathbf{b}^b(t) \quad \mathbf{b}^b(t_0)]|)\mathrm{atan}\left(\frac{\left\|(\mathbf{b}^b(t) \wedge \mathbf{v}_3^b) \wedge (\mathbf{b}^b(t_0) \wedge \mathbf{v}_3^b)\right\|}{(\mathbf{b}^b(t) \wedge \mathbf{v}_3^b) \cdot (\mathbf{b}^b(t) \wedge \mathbf{v}_3^b)}\right) \qquad (17)$$

∧ représentant le produit vectoriel.

Pour déterminer $\beta(t_0)$, il faut une observation de $\beta$ à $t = t_0$ :

- la première solution consiste à avoir une connaissance préalable de l'angle $\beta(t)$, par exemple une valeur critique aux instants de FF, HS, TO, MS, et ZC ;
- la deuxième solution consiste à utiliser des mesures inertielles. L'orientation du capteur de mouvement relative au tibia doit être connue (i.e. un axe du repère BF doit être aligné avec le tibia).

On suppose que $\mathbf{z}_{BF}$ est aligné avec la direction du tibia. Si un accéléromètre est utilisé, ses mesures à l'instant FF de maintien du pied à plat (sur un sol plat) est $a^b(t_0)=\mathbf{g}^b$, $\beta(t_0)$ pouvant être déterminé comme l'angle entre $\mathbf{z}_{BF}$ et la verticale donnée par $a^b(t_0)=\mathbf{g}^b$, $\mathbf{z}_{BF}$ et $a^b(t_0)$ étant tous deux projetés dans le plan de la marche.

$$\beta(t_0) = \mathrm{sign}(|[\mathbf{v}_3^b \quad \mathbf{a}^b(t_0) \quad \mathbf{z}_{BF}^b]|)\mathrm{atan}\left(\frac{\left\|(\mathbf{a}^b(t_0) \wedge \mathbf{v}_3^b) \wedge (\mathbf{z}_{BF}^b \wedge \mathbf{v}_3^b)\right\|}{(\mathbf{a}^b(t_0) \wedge \mathbf{v}_3^b) \cdot (\mathbf{z}_{BF}^b \wedge \mathbf{v}_3^b)}\right) \qquad (18)$$

Après avoir calculé $\beta_r$ et $\beta_l$ en utilisant les relations 16, 17, et 18, on peut procéder à l'estimation de la pose des jambes.

On peut remarquer que les occurrences du maximum de $\beta$ et de $\beta_1$ sont relativement proches (cf figure 13a et 13b). Dans la suite, on suppose que :

- $\beta_1$ atteint son maximum lorsque $\beta$ atteint son maximum (i.e. au passage par zéro de $d\beta/dt$) ;
- $\beta_{r1}$ atteint son minimum lorsque $\beta_{l1}$ atteint son maximum et vice versa.

Soit $t_{ZC}^{\kappa}$ l'instant de passage par zéro de $d\beta/dt$ pour le pas numéro $\kappa$ lorsque $\beta_r$ ou $\beta_l$ atteint un maximum local. $\kappa$ est incrémenté à chaque passage par zéro lorsque $\beta_r$ ou $\beta_l$ atteignent leurs valeur maximale. Dans la suite, on se concentre sur ces moments critiques pour interpoler $\beta_1$.

Pour chaque pas $\kappa$, $\beta_{r1}^{\kappa}$ et $\beta_{l1}^{\kappa}$ sont interpolées avec deux fonctions cosinus, pour $t \in I_{ZC}^{\kappa}$, avec :

- $I_{ZC}^{\kappa} = [t_{l,ZC}^{\kappa-1}, t_{r,ZC}^{\kappa}]$ lorsque $\beta_{r1}$ croît de sa valeur minimum (calculé précédemment au passage par zéro au pas $\kappa$-1) à sa valeur maximum, alors que $\beta_{l1}$ décroît de sa valeur maximum (précédemment calculée au passage par zéro au pas $\kappa$-1) à sa valeur minimum.

- $I_{ZC}^{\kappa} = [t_{r,ZC}^{\kappa-1}, t_{l,ZC}^{\kappa}]$ lorsque $\beta_{r1}$ décroît de sa valeur maximum (précédemment calculée au passage par zéro au pas $\kappa$-1) à sa valeur minimum et $\beta_{l1}$ croît de sa valeur minimum (calculée précédemment au passage par zéro au pas $\kappa$-1) à sa valeur maximum.

La détermination d'une valeur de $\beta_l$ pour un échantillon temporel de chaque intervalle est suffisante pour trouver les paramètres du modèle d'évolution de $\beta_1$. En utilisant seulement les valeurs $\beta_r$ et $\beta_l$, il n'est pas possible de déterminer les valeurs d'angle de $\beta_1$. Par conséquent, des informations ou hypothèses supplémentaires doivent être inclus.

Par exemple, pendant une marche, à $t = t_{ZC}$ on peut considérer que l'angle du genou $\beta_2$ est proche de zéro. Toutefois, les valeurs exactes ne peuvent être que difficilement prédites car ils dépendent beaucoup des paramètres de comportement de la marche (par exemple, la pente de la marche, la longueur du pas, la vitesse, l'utilisateur, etc...)

Il est possible d'estimer $\tilde{\beta}_1(t)$ en modélisant $\beta_1(t)$ pour $t \in [t_{ZC}^{\kappa-1}, t_{ZC}^{\kappa}]$ par une fonction cosinus passant par les valeurs estimées $\beta_1(t_{ZC}^{\kappa-1})$ et $\beta_1(t_{ZC}^{\kappa})$. Ainsi, il n'est pas nécessaire d'avoir un capteur de mouvement pour observer $\beta_1$ en continu. En outre, puisque $\beta = \beta_1 + \beta_2$, on obtient l'angle $\tilde{\beta}_2$ par la relation suivante :

$$\tilde{\beta}_2(t) = \beta(t) - \tilde{\beta}_1(t) \tag{19}$$

Par conséquent, il est possible de calculer l'ensemble des quatre angles $\{\tilde{\beta}_{r1}(t), \tilde{\beta}_{r2}(t), \tilde{\beta}_{l1}(t), \tilde{\beta}_{l2}(t)\}$.

Enfin, les coordonnées des jambes sont calculées comme décrit précédemment, où la phase d'appui ST est commutée entre les jambes gauche et droite à chaque pose du pied au sol HS.

Les coordonnées dans le repère NF lié à la Terre peuvent être calculées, l'angle de cap $\varphi_W^{(l)}$ de la taille (ou hanches) du piéton étant déterminée à partir d'un ou de l'ensemble des magnétomètres montés sur les chevilles.

De manière avantageuse, il est possible d'utiliser deux magnétomètres triaxiaux 3M (ou 2M dans le plan de la marche) ou deux gyromètres triaxiaux 3G (ou 1G dans l'axe orthogonal au plan de la marche) montés sur chaque cheville du piéton. Ensuite, on applique les étapes suivantes :

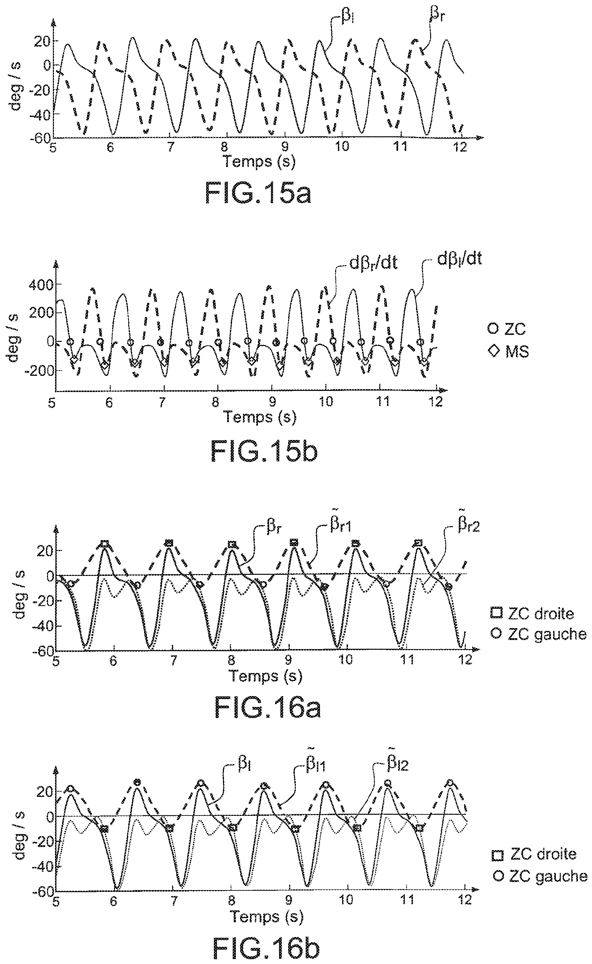

1) les angles $\beta_r$ and $\beta_l$ sont déterminés comme décrit précédemment par les relations 14 et 15 avec un gyromètre ou 16, 17 et 18 avec un magnétomètre. Ces angles sont représentés sur la figure 15a. Comme on peut le remarquer, pour calculer $\beta(t_0)$, les mesures de l'accéléromètre dans la phase d'appui ST (i.e. la mesure de la gravité) est nécessaire pour déterminer la verticale. Cependant, $\beta(t_0)$ est initialisé sans faire référence à la gravité en supposant que $\beta(t_0) = 0$ au début de la marche lorsque le piéton est debout ;
2) les vitesses angulaires $d\beta_r/dt$ et $d\beta_l/dt$ sont calculées (cf figure 15b) ;
3) la détection de pas permettant de détecter les instants HS et ZC est mise en oeuvre (cf figure 15b) ;

4) les angles $\beta_{r1}$ et $\beta_{l1}$ sont modélisés par des fonctions cosinus en s'appuyant sur les occurrences $t_{ZC}^{\kappa-1}$ et $t_{ZC}^{\kappa}$, et sur $\beta(t_{ZC}^{\kappa-1})$ et $\beta(t_{ZC}^{\kappa})$ (cf figure 16a et 16b). En l'espèce, $\beta_2(t_{ZC})$ est fixé à - 4° , aussi $\beta_1(t_{ZC}) = \beta(t_{ZC}) + 4°$ ;
5) les angles $\tilde{\beta}_{r2}$ et $\tilde{\beta}_{l2}$ sont calculés (cf figure 16a et 16b) et la pose des jambes est déterminée.

## Revendications

1.   Procédé de détermination de valeurs de paramètres représentatifs d'un mouvement à l'aide d'un ensemble capteur, le mouvement impliquant deux membres supérieurs ou inférieurs d'un être vivant ou artificiel, ces deux membres étant représentés sous la forme d'une chaîne articulée dans laquelle chaque membre est lui-même représenté par au moins deux segments reliés entre eux par au moins une articulation et lesdits au moins deux membres sont reliés entre eux par une articulation, la chaîne articulée présentant au moins six degrés de liberté, l'ensemble capteur étant fixé à l'être vivant ou artificiel et comportant au moins un capteur, dit capteur d'orientation, de paramètre représentatif d'une orientation d'un premier segment de la chaîne articulée, ce procédé comportant les étapes suivantes :

   - réception d'une valeur d'orientation mesurée et fournie par le capteur d'orientation,

- estimation d'une valeur d'au moins un premier paramètre représentatif d'un mouvement du premier segment par traitement de la valeur d'orientation fournie,

**caractérisé en ce qu'**il comporte en outre les étapes suivantes :

- établissement d'un modèle de mouvement prédéterminé de la chaîne articulée, dit modèle d'évolution temporelle, comportant au moins une relation de dépendance temporelle entre ledit au moins un premier paramètre et au moins un autre paramètre représentatif d'un mouvement d'un autre segment de la chaîne articulée, et
- estimation d'une valeur dudit au moins un autre paramètre par application du modèle d'évolution temporelle à la valeur estimée dudit au moins un premier paramètre.

2. Procédé de détermination selon la revendication 1, dans lequel, le modèle d'évolution temporelle comportant une relation de dépendance temporelle, notamment une relation de décalage temporel, entre les mouvements d'au moins deux membres distincts de l'être vivant ou artificiel, ledit au moins un autre paramètre comporte un paramètre représentatif d'un mouvement d'un autre segment de la chaîne articulée associé à au moins une partie d'un autre membre de la chaîne articulée que le membre représenté au moins par le premier segment.

3. Procédé de détermination selon la revendication 1 ou 2, dans lequel le modèle d'évolution temporelle comporte une relation de dépendance temporelle des orientations de tous les segments de la chaîne articulée relativement à celle du premier segment.

4. Procédé de détermination selon l'une quelconque des revendications 1 à 3, dans lequel :

- chaque membre de l'être vivant ou artificiel est lui-même représenté par deux segments reliés entre eux par une articulation, la chaîne articulée présentant six degrés de liberté, ou
- chaque membre de l'être vivant ou artificiel est lui-même représenté par trois segments reliés entre eux deux à deux par deux articulations, la chaîne articulée présentant huit degrés de liberté.

5. Programme d'ordinateur téléchargeable depuis un réseau de communication et/ou enregistré sur un support lisible par ordinateur et/ou exécutable par un processeur, **caractérisé en ce qu'**il comprend des instructions pour l'exécution des étapes d'un procédé de détermination selon l'une quelconque des revendications 1 à 4, lorsque ledit programme est exécuté sur un ordinateur.

6. Système de détermination de valeurs de paramètres représentatifs d'un mouvement, ce mouvement impliquant deux membres supérieurs ou inférieurs d'un être vivant ou artificiel, ces deux membres étant représentés sous la forme d'une chaîne articulée dans laquelle chaque membre est lui-même représenté par au moins deux segments reliés entre eux par au moins une articulation et lesdits au moins deux membres sont reliés entre eux par une articulation, la chaîne articulée présentant au moins six degrés de liberté, comportant :

- au moins un capteur, dit capteur d'orientation, de paramètre représentatif d'une orientation d'un premier segment de la chaîne articulée, ce capteur d'orientation étant muni de moyens de fixation à une partie du membre représentée par ce premier segment, et
- des moyens de traitement de valeurs d'orientation mesurées et fournies par le capteur d'orientation, conçus pour la mise en oeuvre d'un procédé de détermination selon l'une quelconque des revendications 1 à 4.

7. Système de détermination selon la revendication 6, dans lequel le capteur d'orientation comporte au moins l'un des éléments de l'ensemble constitué d'un magnétomètre à au moins un axe de mesure et d'un gyromètre à au moins un axe de mesure.

8. Système de détermination selon la revendication 6 ou 7, comportant plusieurs capteurs d'orientation, à raison d'au moins un capteur d'orientation par membre de l'être vivant ou artificiel.

9. Système de détermination selon l'une quelconque des revendications 6 à 8, comportant en outre un capteur d'accélération à au moins deux axes de mesure muni de moyens de fixation à la partie du membre représentée par le premier segment, pour l'estimation de valeurs de position cartésienne d'un point lié au premier segment.

10. Système de détermination selon l'une quelconque des revendications 6 à 9, comportant en outre au moins l'un des éléments de l'ensemble constitué de premiers moyens de détection de pose sur le sol d'un pied de l'être vivant ou artificiel représenté par ladite chaîne articulée, de moyens d'estimation d'une distance inter-pieds et de seconds moyens de détection de cycles de déplacement ou d'instants particuliers caractéristiques de ces cycles.

**Patentansprüche**

1. Bestimmungsverfahren der repräsentativen Parameterwerte einer Bewegung mit Hilfe einer Sensorgruppe, wobei die Bewegung zwei obere oder untere Gliedmaßen eines lebendigen oder künstlichen Wesens einbezieht, wobei die beiden Gliedmaßen in der Form einer Gelenkkette repräsentiert werden, in der jede Gliedmaße ihrerseits durch mindestens zwei miteinander durch mindestens ein Gelenk verbundene Segmente repräsentiert wird und die mindestens zwei Gliedmaßen miteinander durch ein Gelenk verbunden sind, wobei die Gelenkkette mindestens sechs Freiheitsgrade aufweist, wobei die Sensorgruppe am lebendigen oder künstlichen Wesen befestigt wird und mindestens einen Sensor, den Ausrichtungssensor, für den repräsentativen Parameter einer Ausrichtung eines ersten Segments der Gelenkkette umfasst, wobei dieses Verfahren die folgenden Schritte umfasst:

   - Empfang eines durch den Ausrichtungssensor gemessenen und bereitgestellten Ausrichtungswertes,
   - Abschätzung eines Wertes von mindestens einem ersten repräsentativen Parameter einer Bewegung des ersten Segments durch Verarbeitung des bereitgestellten Ausrichtungswertes,

   **dadurch gekennzeichnet, dass** es ferner die folgenden Schritte umfasst:

   - Einrichtung eines vorbestimmten Bewegungsmodells der Gelenkkette, das Zeitevolutionsmodell, umfassend mindestens eine zeitabhängige Beziehung zwischen dem mindestens einen ersten Parameter und mindestens einem weiteren repräsentativen Parameter einer Bewegung eines weiteren Segments der Gelenkkette, und
   - Abschätzung eines Wertes des mindestens einen weiteren Parameters durch Anwendung des Zeitevolutionsmodells auf den abgeschätzten Wert des mindestens einen ersten Parameters.

2. Bestimmungsverfahren gemäß Anspruch 1, wobei das Zeitevolutionsmodell eine zeitabhängige Beziehung, insbesondere eine zeitversetzte Beziehung, zwischen den Bewegungen von mindestens zwei verschiedenen Gliedmaßen des lebendigen oder künstlichen Wesens umfasst, und der mindestens eine weitere Parameter einen repräsentativen Parameter einer Bewegung eines weiteren Segments der Gelenkkette, der mit mindestens einem Teil einer weiteren Gliedmaße der Gelenkkette assoziiert ist, außer der durch mindestens das erste Segment repräsentierten Gliedmaße, umfasst.

3. Bestimmungsverfahren gemäß Anspruch 1 oder 2, wobei das Zeitevolutionsmodell eine zeitabhängige Beziehung der Ausrichtungen aller Segmente der Gelenkkette relativ zu der des ersten Segments umfasst.

4. Bestimmungsverfahren gemäß einem der Ansprüche 1 bis 3, wobei:

   - jede Gliedmaße des lebendigen oder künstlichen Wesens seinerseits durch zwei miteinander durch ein Gelenk verbundene Segmente repräsentiert wird, wobei die Gelenkkette sechs Freiheitsgrade aufweist, oder
   - jede Gliedmaße des lebendigen oder künstlichen Wesens seinerseits durch drei miteinander durch zwei Gelenke paarweise verbundene Segmente repräsentiert wird, wobei die Gelenkkette acht Freiheitsgrade aufweist.

5. Aus einem Kommunikationsnetz herunterladbares und/oder auf einem durch einen Computer lesbaren Träger gespeichertes und/oder durch einen Prozessor ausführbares Computerprogramm, **dadurch gekennzeichnet, dass** es Anweisungen zur Ausführung der Schritte eines Bestimmungsverfahrens gemäß einem der Ansprüche 1 bis 4 umfasst, wenn das Programm auf einem Computer ausgeführt wird.

6. Bestimmungssystem der Werte von repräsentativen Parametern einer Bewegung, wobei diese Bewegung zwei obere oder untere Gliedmaßen eines lebendigen oder künstlichen Wesens einbezieht, wobei diese beiden Gliedmaßen in der Form einer Gelenkkette repräsentiert werden, in der jede Gliedmaße ihrerseits durch mindestens zwei miteinander durch mindestens ein Gelenk verbundene Segmente repräsentiert wird und die mindestens zwei Gliedmaßen miteinander durch ein Gelenk verbunden sind, wobei die Gelenkkette mindestens sechs Freiheitsgrade aufweist, umfassend:

   - mindestens einen Sensor, den Ausrichtungssensor, für den repräsentativen Parameter einer Ausrichtung eines ersten Segments der Gelenkkette, wobei der Ausrichtungssensor mit Mitteln zur Befestigung an einen Teil der durch dieses erste Segment repräsentierten Gliedmaße ausgestattet ist, und

- Mittel zur Verarbeitung der durch den Ausrichtungssensor gemessenen und bereitgestellten Ausrichtungswerte, konzipiert für das Umsetzen eines Bestimmungsverfahrens gemäß einem der Ansprüche 1 bis 4.

**7.** Bestimmungssystem gemäß Anspruch 6, wobei der Ausrichtungssensor mindestens eines der Elemente der Gruppe, bestehend aus einem Magnetometer mit mindestens einer Messachse und einem Gyrometer mit mindestens einer Messachse, umfasst.

**8.** Bestimmungssystem gemäß Anspruch 6 oder 7, umfassend mehrere Ausrichtungssensoren, mit mindestens einem Ausrichtungssensor je Gliedmaße des lebendigen oder künstlichen Wesens.

**9.** Bestimmungssystem gemäß einem der Ansprüche 6 bis 8, ferner umfassend einen mit Mitteln zur Befestigung an der durch das erste Segment repräsentierten Gliedmaße ausgestatteten Beschleunigungssensor mit mindestens zwei Messachsen für die Abschätzung kartesischer Positionswerte eines mit dem ersten Segment verbundenen Punkts.

**10.** Bestimmungssystem gemäß einem der Ansprüche 6 bis 9, ferner umfassend mindestens eines der Elemente der Gruppe, bestehend aus ersten Mitteln zur Erfassung der Stellung eines durch die Gelenkkette repräsentierten Fußes des lebendigen oder künstlichen Wesens auf dem Boden, Mitteln zur Abschätzung einer Zwischenfußdistanz und zweiten Mitteln zur Erfassung von Fortbewegungszyklen oder spezieller charakteristischer Zeitpunkte dieser Zyklen.

**Claims**

**1.** Method for determining values of parameters representing a movement by means of a sensor assembly, the movement involving two upper or lower limbs of a living or artificial being, wherein these two limbs are represented in the form of an articulated chain in which each limb is itself represented by at least two segments connected together by at least one articulation and said at least two limbs are connected together by an articulation, wherein the articulated chain has at least six degrees of freedom, the sensor assembly being fixed to the living or artificial being and comprising at least one sensor, referred to as the orientation sensor, for a parameter representing an orientation of a first segment of the articulated chain, this method comprising the following steps:

- reception of an orientation value measured and supplied by the orientation sensor,
- estimation of a value of at least one first parameter representing a movement of the first segment by processing of the orientation value supplied,

**characterised in that** it also comprises the following steps:

- establishing a predetermined movement model of the articulated chain, referred to as the time-change model, comprising at least one relationship of time dependency between said at least one first parameter and at least one other parameter representing a movement of another segment of the articulated chain, and
- estimation of a value of said at least one other parameter by application of the time-change model to the estimated value of said at least one first parameter.

**2.** Determination method according to claim 1, wherein, the time change model comprising a relationship of time dependency, in particular a relationship of time offset, between the movements of at least two distinct limbs of the living or artificial being, said at least one other parameter comprises a parameter representing a movement of another segment of the articulated chain associated with at least part of a limb of the articulated chain other than the limb represented at least by the first segment.

**3.** Determination method according to claim 1 or 2, wherein the time-change model comprises a relationship of time dependency of the orientations of all the segments of the articulated chain relative to that of the first segment.

**4.** Determination method according to any one of claims 1 to 3, wherein:

- each limb of the living or artificial being is itself represented by two segments connected together by an articulation, the articulated chain having six degrees of freedom, or

- each limb of the living or artificial being is itself represented by three segments connected together in pairs by two articulations, the articulated chain having eight degrees of freedom.

5. Computer program downloadable from a communication network and/or recorded on a medium readable by computer and/or executable by a processor, **characterised in that** it comprises instructions for executing the steps of a determination method according to any one of claims 1 to 4, when said program is executed on a computer.

6. System for determining values of parameters representing a movement, this movement involving two upper or lower limbs of a living or artificial being, wherein these two limbs are represented in the form of an articulated chain in which each limb is itself represented by at least two segments connected together by at least one articulation and said at least two limbs are connected together by an articulation, wherein the articulated chain has at least six degrees of freedom, comprising:

   - at least one sensor, referred to as the orientation sensor, for a parameter representing an orientation of a first segment of the articulated chain, this orientation sensor being provided with means for fixing to a part of the limb represented by this first segment, and
   - means for processing orientation values measured and supplied by the orientation sensor, designed for implementing a determination method according to any one of claims 1 to 4.

7. Determination system according to claim 6, wherein the orientation sensor comprises at least one of the elements of the assembly consisting of a magnetometer with at least one measurement axis and a gyrometer with at least one measurement axis.

8. Determination system according to claim 6 or 7, comprising several orientation sensors, with at least one orientation sensor per limb of the living or artificial being.

9. Determination system according to any one of claims 6 to 8, further comprising an acceleration sensor with at least two measurement axes provided with means for fixing the part of the limb represented by the first segment, for estimating cartesian position values of a point linked to the first segment.

10. Determination system according to any one of claims 6 to 9, further comprising at least one of the elements of the set consisting of first means for detecting the placing on the ground of a foot of the living or artificial being represented by said articulated chain, means for estimating an inter-foot distance and second means for detecting movement cycles or particular instants characteristic of these cycles.

FIG.1

FIG.2

FIG.3

FIG.5a

FIG.5b

FIG.5c

FIG.6

FIG.7a

FIG.7b

FIG.8a

FIG.8b

FIG.9a

FIG.9b

FIG.9c

FIG.9d

FIG.10a

FIG.10b

28

FIG.12a

FIG.12b

FIG.12c

FIG.12d

FIG.12e

FIG.13a

FIG.13b

FIG.14a

FIG.14b

FIG.14c

FIG.14d

FIG.14e

FIG.14f

FIG.15a

FIG.15b

FIG.16a

FIG.16b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20080285805 A
- EP 1984696 A
- EP 1990138 A1
- US 7467060 B
- WO 2010046364 A
- US 20020002863 A
- EP 2106747 A

**Littérature non-brevet citée dans la description**

- **Q. LADETTO ; B. MERMINOD.** In step with INS: navigation for the blind, tracking emergency crews. *GPS World,* 2002, vol. 13 (10), 30-38
- **S. BONNET ; R. HELIOT.** A Magnetometer-Based approach for studying human movements. *IEEE Transactions on Biomédical Engineering,* 2007, vol. 54 (7), 1353-1355
- **H.-J. JANG ; J.W. KIM ; D.H. HWANG.** Robust step détection method for pedestrian navigation systems. *Electronics Letters,* 2007, vol. 43 (14